# EUROPEAN PATENT APPLICATION

(11) **EP 3 494 998 A1**
(43) Date of publication of application: **12.06.2019**
(21) Application number: 17206498.2
(22) Date of filing: 11.12.2017
(51) Int. Cl.: A61K 51/04

(54) **GLYCOSYLATED PSMA INHIBITORS FOR IMAGING AND ENDORADIOTHERAPY**

(71) Applicant: Technische Universität München, 80333 München (DE)
(72) Inventor: The designation of the inventor has not yet been filed
(74) Representative: Vossius & Partner Patentanwälte Rechtsanwälte mbB

(57) **Abstract**

The present disclosure relates to imaging and endoradiotherapy of diseases involving prostate-specific membrane antigen (PSMA). Provided are compounds which bind or inhibit PSMA and furthermore carry at least one moiety which is amenable to radiolabeling. Provided are also medical uses of such compounds.

## Description

The present disclosure relates to imaging and endoradiotherapy of diseases involving prostate-specific membrane antigen (PSMA). Provided are compounds which bind or inhibit PSMA and furthermore carry at least one moiety which is amenable to radiolabeling. Provided are also medical uses of such compounds.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Prostate Cancer (PCa) remained over the last decades the most common malignant disease in men with high incidence for poor survival rates. Due to its overexpression in prostate cancer (Silver, D.A., et al., Prostate-specific membrane antigen expression in normal and malignant human tissues. Clinical Cancer Research, 1997. 3(1): p. 81-85), prostate-specific membrane antigen (PSMA) or glutamate carboxypeptidase II (GCP II) proved its eligibility as excellent target for the development of highly sensitive radiolabeled agents for endoradiotherapy and imaging of PCa (Afshar-Oromieh, A., et al., The diagnostic value of PET/CT imaging with the 68Ga-labelled PSMA ligand HBED-CC in the diagnosis of recurrent prostate cancer. European journal of nuclear medicine and molecular imaging, 2015. 42(2): p. 197-209; Benešová, M., et al., Preclinical Evaluation of a Tailor-Made DOTA-Conjugated PSMA Inhibitor with Optimized Linker Moiety for Imaging and Endoradiotherapy of Prostate Cancer. Journal of Nuclear Medicine, 2015. 56(6): p. 914-920; Robu, S., et al., Preclinical evaluation and first patient application of 99mTc-PSMA-I&S for SPECT imaging and radioguided surgery in prostate cancer. Journal of Nuclear Medicine, 2016: p. jnumed. 116.178939; Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083; Rowe, S., et al., PET imaging of prostate-specific membrane antigen in prostate cancer: current state of the art and future challenges. Prostate cancer and prostatic diseases, 2016; Maurer, T., et al., Current use of PSMA-PET in prostate cancer management. Nature Reviews Urology, 2016). Prostate-specific membrane antigen is an extracellular hydrolase whose catalytic center comprises two zinc(II) ions with a bridging hydroxido ligand. It is highly upregulated in metastatic and hormone-refractory prostate carcinomas, but its physiologic expression has also been reported in kidneys, salivary glands, small intestine, brain and, to a low extent, also in healthy prostate tissue. In the intestine, PSMA facilitates absorption of folate by conversion of pteroylpoly-γ-glutamate to the pteroylglutamate (folate). In the brain, it hydrolyses N-acetyl-Laspartyl- L-glutamate (NAAG) to N-acetyl-L-aspartate and glutamate. The enzymatic function of PSMA in normal and diseased prostate has yet not been clarified.

PSMA targeting molecules usually comprise a binding unit that encompasses a zinc-binding group (such as urea (Zhou, J., et al., NAAG peptidase inhibitors and their potential for diagnosis and therapy. Nature Reviews Drug Discovery, 2005. 4(12): p. 1015-1026), phosphinate or phosphoramidate) connected to a P1' glutamate moiety, which warrants high affinity and specificity to PSMA and is typically further connected to an effector functionality (Machulkin, A.E., et al., Small-molecule PSMA ligands. Current state, SAR and perspectives. Journal of drug targeting, 2016: p. 1-15). The effector part is more flexible and to some extent tolerant towards structural modifications. The entrance tunnel of PSMA accommodates two other prominent structural features, which are important for ligand binding. The first one is an arginine patch, a positively charged area at the wall of the entrance funnel and the structural explanation for the preference of negatively charged functionalities at the P1 position of PSMA. Upon binding the concerted repositioning of the arginine side chains can lead to the opening of an S1 hydrophobic accessory pocket, the second important structure, that has been shown to accommodate an iodo-benzyl group of several urea based inhibitors, thus contributing to their high affinity for PSMA (Barinka, C., et al., Interactions between Human Glutamate Carboxypeptidase II and Urea-Based Inhibitors: Structural Characterization†. Journal of medicinal chemistry, 2008. 51(24): p. 7737-7743).

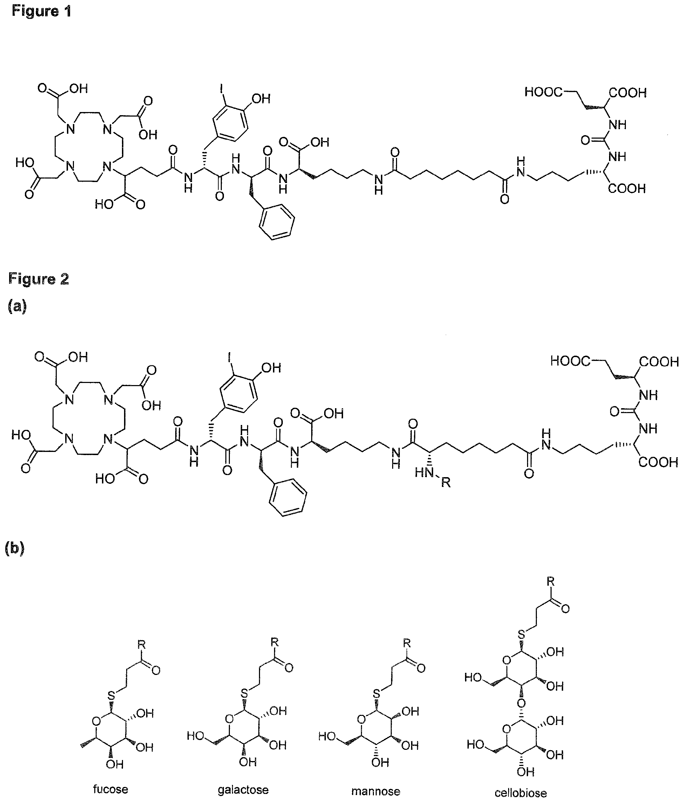

Zhang et al. discovered a remote binding site of PSMA, which can be employed for bidentate binding mode (Zhang, A.X., et al., A remote arene-binding site on prostate specific membrane antigen revealed by antibody-recruiting small molecules. Journal of the American Chemical Society, 2010. 132(36): p. 12711-12716). The so called arene-binding site is a simple structural motif shaped by the side chains of Arg463, Arg511 and Trp541, and is part of the PSMA entrance lid. The engagement of the arene-binding site by a distal inhibitor moiety can result in a substantial increase in the inhibitor affinity for PSMA due to avidity effects. PSMA I&T (see Figure 1) was developed with the intention to interact this way with PSMA, albeit no crystal structure analysis of binding mode is available. A necessary feature according to Zhang et al. is a linker unit (suberic acid in the case of PSMA I&T) which facilitates an open conformation of the entrance lid of PSMA and thereby enabling the accessibility of the arene-binding site. It was further shown that the structural composition of the linker has a significant impact on the tumor-targeting and biologic activity as well as on imaging contrast and pharmacokinetics (Liu, T., et al., Spacer length effects on in vitro imaging and surface accessibility of fluorescent inhibitors of prostate specific membrane antigen. Bioorganic & medicinal chemistry letters, 2011. 21(23): p. 7013-7016), properties which are crucial for both high imaging quality and efficient targeted endoradiotherapy.

Two categories of PSMA targeting inhibitors are currently used in clinical settings. On the one side are tracer with chelating units for radionuclide complexation as PSMA I&T or related compounds (Kiess, A.P., et al., Prostate-specific membrane antigen as a target for cancer imaging and therapy. The quarterly journal of nuclear medicine and molecular imaging: official publication of the Italian Association of Nuclear Medicine (AIMN)[and] the International Association of Radiopharmacology (IAR),[and] Section of the Society of... 2015. 59(3): p. 241). On the other side are small molecules, comprising a targeting unit and effector molecules. Depending on the used radionuclide/halogen, the radiolabeled PSMA inhibitors may be used for imaging or endoradiotherapy. Among small molecule inhibitors with chelators for imaging, the most often used agents for selective PSMA imaging are PSMA HBED-CC (Eder, M., et al., 68Ga-complex lipophilicity and the targeting property of a urea-based PSMA inhibitor for PET imaging. Bioconjugate chemistry, 2012. 23(4): p. 688-697), PSMA-617 (Benešová, M., et al., Preclinical Evaluation of a Tailor-Made DOTA-Conjugated PSMA Inhibitor with Optimized Linker Moiety for Imaging and Endoradiotherapy of Prostate Cancer. Journal of Nuclear Medicine, 2015. 56(6): p. 914-920) and PSMA I&T (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083). PSMA HBED-CC, or PSMA-11 was one the first PSMA inhibitors and is currently used for imaging since therapeutic applications are not possible with the chelator HBED-CC. However, due to the unique physical characteristics and the advantages of ¹⁸F for PET imaging, like the longer half-life, the low positron energy, which results in higher image resolution and the possibility for largescale production in a cyclotron, several groups have focused on the development of ¹⁸F-labeled urea-based Inhibitors for PCa imaging. The ¹⁸F-labeled urea-based PSMA inhibitor [¹⁸F]DCFPyl demonstrated promising results in detection of primary and metastatic PCa (Rowe, S.P., et al., PSMA-Based [*8F] DCFPyL PET/CT Is Superior to Conventional Imaging for Lesion Detection in Patients with Metastatic Prostate Cancer. Molecular Imaging and Biology, 2016: p. 1-9) and superiority to [⁶⁸Ga]PSMA-HBED-CC in a comparative study (Dietlein, M., et al., Comparison of [18F] DCFPyL and [68Ga] Ga-PSMA-HBED-CC for PSMA-PET imaging in patients with relapsed prostate cancer. Molecular Imaging and Biology, 2015. 17(4): p. 575-584).

PSMA DKFZ 617 (Benešová, M., et al., Preclinical Evaluation of a Tailor-Made DOTA-Conjugated PSMA Inhibitor with Optimized Linker Moiety for Imaging and Endoradiotherapy of Prostate Cancer. Journal of Nuclear Medicine, 2015. 56(6): p. 914-920, Becker, A., et al., Nephro-and hepatotoxicity after radioligand therapy of metastatic castrate-resistant prostate cancer with 177Lu-PSMA-617. Journal of Nuclear Medicine, 2016. 57(supplement 2): p. 1430-1430;Rahbar, K., et al., Response and tolerability of a single dose of 177Lu-PSMA-617 in patients with metastatic castration-resistant prostafe cancer: a multicenter retrospective analysis. Journal of Nuclear Medicine, 2016: p. jnumed. 116.173757) and PSMA I&T (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083, Eiber, M., et al., Systemic radioligand therapy with 177Lu-PSMA I&T in patients with metastatic castration-resistant prostate cancer. Journal of Nuclear Medicine, 2016. 57(supplement 2): p. 61-61; Schottelius, M., et al., [111In] PSMA-I&T: expanding the spectrum of PSMA-I&T applications towards SPECT and radioguided surgery. EJNMMI research, 2015. 5(1): p. 1) are applied in clinical settings for palliative treatment of prostate cancer patients. The chelating unit DOTA and the related DOTAGA, allow not only imaging but also therapeutical applications, since the scope for possible radiometal chelation encompasses ¹¹¹In, ¹⁷⁷Lu, ⁹⁰Y and ²¹³Bi amongst others. [¹¹¹In]PSMA I&T was already clinically implemented for radioguided surgery to assist the surgeon during excision of the malignant tissue (Schottelius, M., et al., [111In] PSMA-I&T: expanding the spectrum of PSMA-I&T applications towards SPECT and radioguided surgery. EJNMMI research, 2015. 5(1): p. 1). Likewise, the recent developed and clinically tested PSMA Inhibitor PSMA I&S (imaging and surgery) demonstrated highly encouraging results (Robu, S., et al., Preclinical evaluation and first patient application of 99mTc-PSMA-I&S for SPECT imaging and radioguided surgery in prostate cancer. Journal of Nuclear Medicine, 2016: p. jnumed. 116.178939).

Endoradiotherapeutic approaches with [¹⁷⁷Lu]PSMA I&T demonstrated efficiency, tolerability and high safety potential in patients receiving up to four cycles with 7.4 GBq. The obtained dosimetric values for organ radiation revealed, that especially the kidneys and the salivary glands receive the highest dose after tumor lesions. Similar radiation values were shown for PSMA DKFZ 617 and [¹⁸F]DCFPyL (Rowe, S.P., et al., PSMA-Based [18F] DCFPyL PET/CT Is Superior to Conventional Imaging for Lesion Detection in Patients with Metastatic Prostate Cancer. Molecular Imaging and Biology, 2016: p. 1-9; Delker, A., et al., Dosimetry for 177Lu-DKFZ-PSMA-617: a new radiopharmaceutical for the treatment of metastatic prostate cancer. European journal of nuclear medicine and molecular imaging, 2016. 43(1): p. 42-51; Kabasakal, L., et al., Pre-therapeutic dosimetry of normal organs and tissues of 177Lu-PSMA-617 prostate-specific membrane antigen (PSMA) inhibitor in patients with castration-resistant prostate cancer. European journal of nuclear medicine and molecular imaging, 2015. 42(13): p. 1976-1983; Yadav, M.P., et al., 177Lu-DKFZ-PSMA-617 therapy in metastatic castration resistant prostate cancer: safety, efficacy, and quality of life assessment. European Journal of Nuclear Medicine and Molecular Imaging, 2016: p. 1-11). These elevated numbers are explainable by the physiologic expression of PSMA (Silver, D.A., et al., Prostate-specific membrane antigen expression in normal and malignant human tissues. Clinical Cancer Research, 1997. 3(1): p. 81-85) and the renal excretion of the radiolabeled compound. The occasional occurring renal and hematological toxicities after administration are usually reversible, although is legitimate concern about chronic toxicity especially in patients with overall long survival rates, like in PCa patients. Therefore, a suitable concept is needed to reduce the unwanted radiation.

In view of the above, the technical problem underlying the present invention can be seen in the provision of means and methods of alleviating radiation-induced side effects of PSMA-targeting radiolabeled diagnostics and therapeutics. More generally speaking, the technical problem can be seen in the provision of improved PSMA binding agents.

This technical problem is solved by the subject-matter summarized in the attached claims and explained in further detail below.

In particular, the present invention provides in a first aspect a compound of formula (I), or a pharmaceutically acceptable salt thereof, 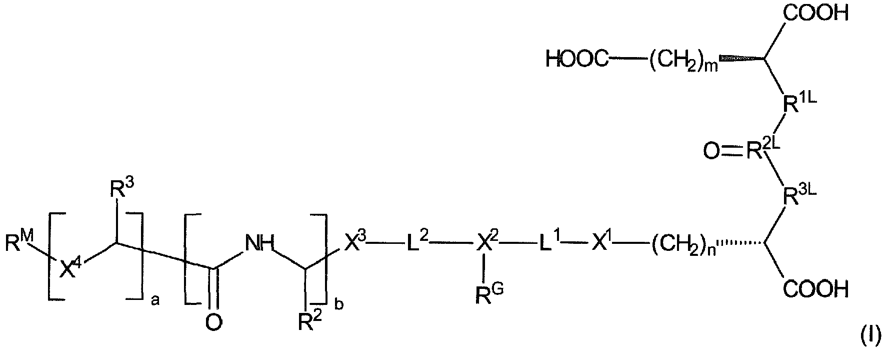 wherein:
- m: is an integer of 2 to 6, preferably 2 to 4, more preferably 2;
- n: is an integer of 2 to 6, preferably 2 to 4, more preferably 4;
- R^{1L}: is CH₂, NH or O, preferably NH;
- R^{2L}: is C or P(OH), preferably C;
- R^{3L}: is CH₂, NH or O, preferably NH;
- X¹: is selected from an amide bond, an ether bond, a thioether bond, an ester bond, a thioester bond, a urea bridge, and an amine bond; and is preferably an amide bond;
- L¹: is an alkanediyl group, preferably a C2-C10 alkanediyl group, more preferably a C3-C6 alkanediyl group, and most preferably a C5 alkanediyl group, which alkanediyl group is optionally substituted by a group selected from -COOH and -NH₂;
- X²: is a trivalent coupling group carrying the substituent R^{G};
- L²: is an alkanediyl group, preferably a C2-C10 alkanediyl group, more preferably a C3-C6 alkanediyl group, and most preferably a C5 alkanediyl group, said alkanediyl group being optionally substituted by a group selected from -COOH and -NH₂;
- X³: is selected from an amide bond, an ether bond, a thioether bond, an ester bond, a thioester bond, a urea bridge, and an amine bond, and is preferably an amide bond;
- a: is 0 or 1, preferably 1;
- b: is 0 or 1, preferably 1;
- R²: is an optionally substituted aryl group or an optionally substituted aralkyl group, which aryl group or aralkyl group may be substituted on its aromatic ring with a substituent selected from halogen, preferably I, and -OH;
- R³: is an optionally substituted aryl group or an optionally substituted aralkyl group, which aryl group or aralkyl group may be substituted on its aromatic ring with a substituent selected from halogen, preferably I, and -OH;
- X⁴: is selected from an amide bond, an ether bond, a thioether bond, an ester bond, a thioester bond, a urea bridge, an amine bond, a group of the formula 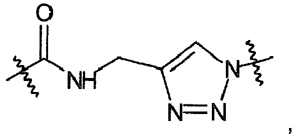 wherein the marked bond at the carbonyl group attaches X⁴ to R^{M} and the other marked bond attaches X⁴ to the remainder of the molecule, and a group of the formula 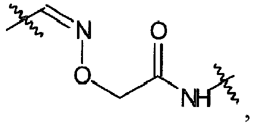 wherein the marked bond at the imino group attaches X⁴ to R^{M} and the other marked bond attaches X⁴ to the remainder of the molecule; and is preferably an amide bond;
- R^{M}: is a marker group which comprises at least one of
(i) a chelating group optionally containing a chelated non-radioactive or radioactive cation, and
(ii) a radiotracer group containing a covalently bound fluorine atom which can be labeled with ¹⁸F by isotopic exchange of ¹⁹F by ¹⁸F or which is labeled with ¹⁸F; and
- R^{G}: is a group of the formula (IIa) or (IIb): 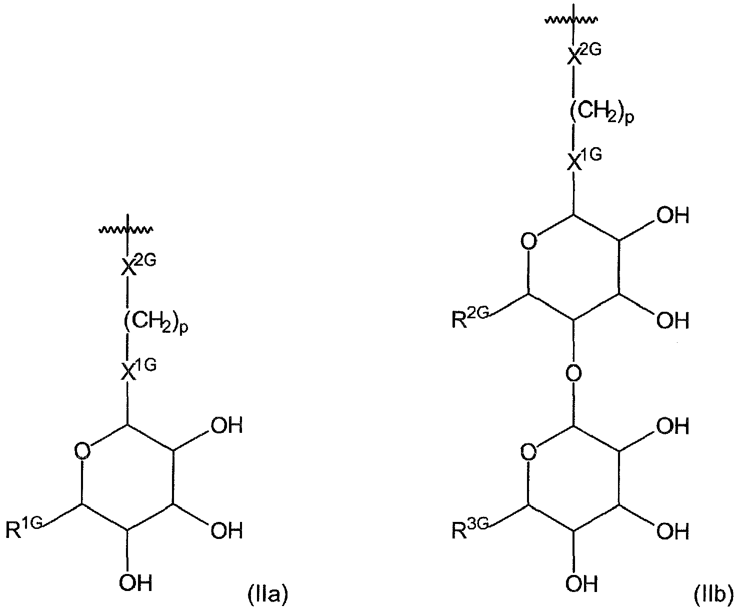 wherein
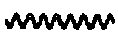 marks the bond which attaches R^{G} to X²;
p is an integer of 1 to 5, preferably 2 or 3, and more preferably 2;
R^{1G} is selected from -OH and -CH₂-OH;
R^{2G} is selected from -OH and -CH₂-OH, and is preferably -CH₂-OH;
R^{3G} is selected from -OH and -CH₂-OH, and is preferably -CH₂-OH;
X^{1G} is selected from S and O, and is preferably S;
X^{2G} is selected from an amide bond, an ether bond, a thioether bond, an ester bond, a thioester bond, an urea bridge, and an amine bond, and is preferably an amide bond.

Similar to PSMA I&T (discussed further above and depicted in Figure 1), which may be viewed as a parent compound, the compounds in accordance with the first aspect comprise at least one site which is amenable to radiolabeling for diagnostic and/or therapeutic purposes. This site is the chelating group.

Deviant from PSMA I&T, the part of the linker connecting the chelating group and the PSMA-binding head group at the two ends of the compound is not linear or essential linear, but instead, and owing to the presence of the carbohydrate, branched.

This structural feature has to be properly gauged in view of the knowledge about the structure of the target molecule PSMA. As noted in the background section herein above, the entrance tunnel of PSMA which leads to the binding site for the head group has a funnel shape. This leads to the expectation that linear linkers in PSMA ligands should be used and that any branched linker would, owing to steric clashes, dramatically reduce or entirely abolish binding. Surprisingly, and contrary to all expectations, the opposite turned out to be the case. In particular, and as can be seen from the examples enclosed herewith, the introduction of carbohydrate moieties in the linear part of the linker allows to maintain or even increase affinity to PSMA. There was no expectation at all that the structure of PSMA would allow for the incorporation of hydrophilic bulky moieties as carbohydrates.

In this regard, reference is made to Bouvet et al. (Targeting Prostate-Specific Membrane Antigen (PSMA) with F-18-Labeled Compounds: the Influence of Prosthetic Groups on Tumor Uptake and Clearance Profile. Molecular Imaging and Biology, 2017: p. 1-10). Their PSMA ligand exhibited only moderate binding to PSMA, while every other compound, modified with an aromatic residue, was found to be significantly more affine.

Having said that, maintaining or even increasing the affinity to the target is not the only advantage of a carbohydrate moiety in a compound in accordance with the first aspect. Rather, glycosylation is a means of modulating pharmacokinetics. The increasing hydrophilicity provides for a more favorable pharmacokinetics.

In particular, the compounds of the invention exhibit accelerated tissue clearance and thus lower activity uptake in PSMA-unspecific tissue. As a consequence, improved tumor-to-tissue ratios are obtained with the glycosylated compound of the invention.

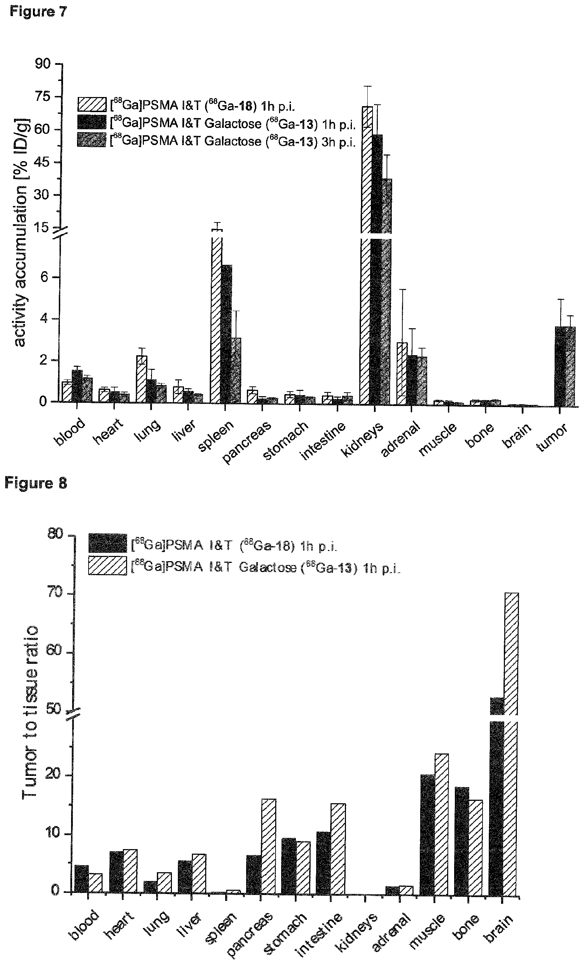

In more detail, sugar- or carbohydrate conjugation of peptides improves the urinary excretion of peptides by shifting the clearance route from hepatobiliary to renal excretion. In addition, they act as non-charged pharmacokinetic modifiers, thus offering the advantage of higher hydrophilicity while avoiding the negative effects of positively or negatively charged moieties (unspecific retention in kidneys, vasculature, or other tissues). Evidence in that respect is provided in Figure 8.

Sugar moieties conjugated to peptides can also reduce plasma protein binding and liver uptake. The advantages of carbohydration of peptide radioligands was recently demonstrated and reviewed for carbohydrated ligands of the somatostatin-2 receptor (sst2) (Cancer Biother Radiopharm. 2004 Apr;19(2):231-44. Radiolabeled carbohydrated somatostatin analogs: a review of the current status. Wester HJ, Schottelius M, Poethko T, Bruus-Jensen K, Schwaiger M.)

Low plasma protein binding might be beneficial in terms of low background activity for PET imaging quality with earlier acquisition after i.v. injection and reduced radiation doses during endoradiotherapy since radiolabeled PSMA tracer are retained at the target site. A higher unbound fraction in the blood pool should results in a faster plasma clearance, if the renal clearance into the bladder is considered as the primarily elimination process.

The introduction of the carbohydrates decreased the albumin binding drastically when compared to the parent compound PSMA I&T. This feature results in faster background clearance (lower radiation) *in vivo;* see the enclosed examples.

Yet further, the compounds provide for high imaging quality.

Further advantages include a reduced uptake in those tissues where PSMA is not expressed or only expressed in low amounts, especially kidneys and salivary glands. This latter effect provides for a significant reduction of radiation-induced side effects occurring in non-target tissues.

Moreover, and since the carbohydrates are highly hydrophilic residues, they are able to compensate high lipophilicity arising from lipophilic modifications e.g. with fluorescence dyes.

Also, carbohydrates remodel the clearance route from hepatic to renal excretion, which is beneficial for therapeutic applications with more lipophilic ligands which are more susceptible for hepatic clearance.

As noted above, salts of the compounds of the invention including compounds of formula (I) (and including their preferred embodiments) are also suitable for use in the context of the invention. It will be understood that these salts are generally pharmaceutically acceptable salt forms of these compounds which may be formed, e.g., by protonation of an atom carrying an electron lone pair which is susceptible to protonation, such as an amino group, with an inorganic or organic acid, or as a salt of a carboxylic acid group with a physiologically acceptable cation as they are well known in the art. Exemplary base addition salts comprise, for example, alkali metal salts such as sodium or potassium salts; alkaline-earth metal salts such as calcium or magnesium salts; ammonium salts; aliphatic amine salts such as trimethylamine, triethylamine, dicyclohexylamine, ethanolamine, diethanolamine, triethanolamine, procaine salts, meglumine salts, diethanol amine salts or ethylenediamine salts; aralkyl amine salts such as N,N-dibenzylethylenediamine salts, benetamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts or lysine salts. Exemplary acid addition salts comprise, for example, mineral acid salts such as hydrochloride, hydrobromide, hydroiodide, sulfate salts, nitrate salts, phosphate salts (such as, e.g., phosphate, hydrogenphosphate, or dihydrogenphosphate salts), carbonate salts, hydrogencarbonate salts or perchlorate salts; organic acid salts such as acetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate or ascorbate salts; sulfonate salts such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate salts; and acidic amino acid salts such as aspartate or glutamate salts.

Further examples of pharmaceutically acceptable salts include, but are not limited to, acetate, adipate, alginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxyethanesulfonate, hydroxynaphthoate, iodide, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, picrate, pivalate, polygalacturonate, propionate, salicylate, stearate, sulfate, subacetate, succinate, tannate, tartrate, teoclate, tosylate, triethiodide, undecanoate, valerate, and the like (see, for example, S. M. Berge et al., "Pharmaceutical Salts", J. Pharm. Sci., 66, pp. 1-19 (1977)).

It is understood that throughout the present specification the term "compound" encompasses solvates, polymorphs, prodrugs, codrugs, cocrystals, tautomers, racemates, enantiomers, or diastereomers or mixtures thereof unless mentioned otherwise.

When the compounds of the present invention are provided in crystalline form, the structure can contain solvent molecules. The solvents are typically pharmaceutically acceptable solvents and include, among others, water (hydrates) or organic solvents. Examples of possible solvates include ethanolates and iso-propanolates.

The term "codrug" refers to two or more therapeutic compounds bonded via a covalent chemical bond. A detailed definition can be found, e.g., in N. Das et al., European Journal of Pharmaceutical Sciences, 41, 2010, 571-588.

The term "cocrystal" refers to a multiple component crystal in which all components are solid under ambient conditions when in their pure form. These components co-exist as a stoichiometric or non-stoichometric ratio of a target molecule or ion (i.e., compound of the present invention) and one or more neutral molecular cocrystal formers. A detailed discussion can be found, for example, in Ning Shan et al., Drug Discovery Today, 13(9/10), 2008, 440-446 and in D. J. Good et al., Cryst. Growth Des., 9(5), 2009, 2252-2264.

The compounds of the present invention can also be provided in the form of a prodrug, namely a compound which is metabolized in vivo to the active metabolite. Suitable prodrugs are, for instance, esters. Specific examples of suitable groups are given, among others, in US 2007/0072831 in paragraphs [0082] to [0118] under the headings prodrugs and protecting groups.

To the extent compounds of the invention exhibit a pH-dependent charged state, it is understood that all possible charged states are embraced. A preferred pH range in this regard is from 0 to 14.

To the extent a compound according to the invention bears a net charge, it is understood that the compound is provided in electroneutral form. This is achieved by one or more counterions, preferred counterions being defined in relation to the term "salt" herein above.

In formula (I), m is an integer of 2 to 6. Preferably, m is 2 to 4, more preferably 2. R^{1L} is CH₂, NH or O, preferably NH. R^{2L} is C or P(OH), preferably C. R^{3L} is CH₂, NH or O, preferably NH. Thus, compounds of formula (I) or their salts are also preferred wherein m is 2, R^{1L} is NH, R^{2L} is C, and R^{3L} is NH.

n is an integer of 2 to 6, preferably 2 to 4, more preferably 2 or 4.

Thus, compounds of formula (I) or their salts are particularly preferred wherein m is 2, n is 2 or 4, R^{1L} is NH, R^{2L} is C, and R^{3L} is NH. More preferred are compounds of formula (I) or their salts wherein m is 2, n is 4, R^{1L} is NH, R^{2L} is C, and R^{3L} is NH.

X¹ in formula (I) is selected from an amide bond (i.e. -C(O)-NH-), an ether bond (i.e. -O-), a thioether bond (i.e. -S-), an ester bond (i.e. -C(O)-O-, a thioester bond (i.e. -C(S)-O- or -C(O)-S-), a urea bridge (i.e. -NH-C(O)-NH-), and an amine bond (i.e. -NH-). Preferred as X¹ is the amide bond. More preferred as X¹ is the amide bond with the nitrogen atom of -C(O)-NH- being attached to the group -(CH₂)ₙ- in formula (I), wherein n is 4. Also more preferred as X¹ is the amide bond with the carbon atom of -C(O)-NH- being attached to the group -(CH₂)ₙ- in formula (I), wherein n is 2.

X³ in formula (I) is selected from an amide bond (i.e. -C(O)-NH-), an ether bond (i.e. -O-), a thioether bond (i.e. -S-), an ester bond (i.e. -C(O)-O-, a thioester bond (i.e. -C(S)-O- or - C(O)-S-), a urea bridge (i.e. -NH-C(O)-NH-), and an amine bond (i.e. -NH-). Preferred as X³ is the amide bond, and more preferred as X³ is the amide bond with the nitrogen atom of -C(O)-NH- being attached to L² in formula (I).

Thus, it is preferred for the compounds of formula (I) or their salts that X¹ and X³ both represent an amide bond, and it is more preferred that X¹ is an amide bond with the nitrogen atom of -C(O)-NH- being attached to the group -(CH₂)ₙ- and X³ is an amide bond with the nitrogen atom of -C(O)-NH- being attached to L².

L¹ in formula (I) is an alkanediyl group, preferably a C2-C10 alkanediyl group, more preferably a C3-C6 alkanediyl group, and most preferably a C3 or C5 alkanediyl group, which alkanediyl group is optionally substituted by a group selected from -COOH and -NH₂. As will be understood, the term "alkanediyl" is used in line with established nomenclature to designate a divalent saturated hydrocarbon group which may be linear or branched. The alkanediyl group, including the preferred embodiments thereof with a defined number of carbon atoms, is preferably a linear alkanediyl group, which may also be represented by the formula -(CH₂)ₓ-, wherein x is an integer corresponding to the number of carbon atoms and, in line with the above, is preferably 2-10, more preferably 3-6, and most preferably 3 or 5.

The alkanediyl group represented by L¹ is optionally substituted by a group selected from -COOH and -NH₂. It will be understood that this applies also for the preferred alkanediyl groups with a defined number of carbon atoms (which does not include the carbon atom of an optional -COOH substituent) and/or the linear structure. It will be also be understood that this includes the possibility that one or more than one, e.g. 2 or 3, substituent(s) selected from -COOH and -NH₂ is (are) attached to L¹. However, it is preferred that L¹ is non-substituted.

L² in formula (I) is an alkanediyl group, preferably a C2-C10 alkanediyl group, more preferably a C3-C6 alkanediyl group, and most preferably a C5 alkanediyl group, which alkanediyl group is optionally substituted by a group selected from -COOH and -NH₂. The alkanediyl group, including the preferred embodiments thereof with a defined number of carbon atoms, is preferably a linear alkanediyl group, which may also be represented by the formula -(CH₂)ₓ-, wherein x is an integer corresponding to the number of carbon atoms and, in line with the above, is preferably 2-10, more preferably 3-6, and most preferably 5.

The alkanediyl group represented by L² is optionally substituted by a group selected from -COOH and -NH₂, preferably with a group -COOH. It will be understood that this applies also for the preferred alkanediyl groups with a defined number of carbon atoms (which does not include the carbon atom of an optional -COOH substituent) and/or the linear structure. It will be also be understood that this includes the possibility that one or more than one, e.g. 2 or 3, substituent(s) selected from -COOH and -NH₂ is (are) attached to L². However, it is preferred that L² carries one substituent which is a -COOH group. In a more preferred embodiment, L² carries one substituent which is a -COOH group and which is present on the carbon atom attached to X³.

It is particularly preferred that L² is a group of the formula -CH(COOH)-(CH₂)₄-, with the carbon atom carrying the substituent -COOH being attached to X³ in formula (I).

R^{G} in formula (I) is a group of the formula (IIa) or (IIb): 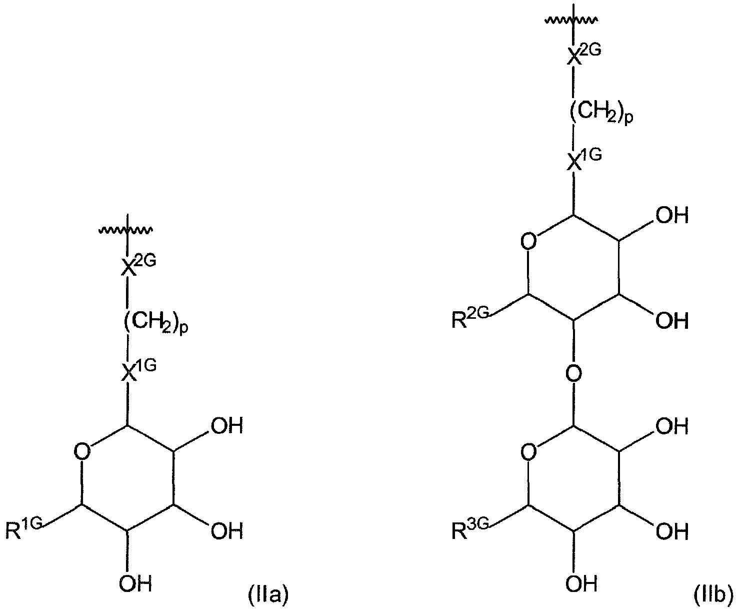 wherein
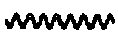 marks the bond which attaches R^{G} to X²;
p is an integer of 1 to 5, preferably 2 or 3, and more preferably 2;
R^{1G} is selected from -OH and -CH₂-OH;
R^{2G} is selected from -OH and -CH₂-OH, and is preferably -CH₂-OH;
R^{3G} is selected from -OH and -CH₂-OH, and is preferably -CH₂-OH;
X^{1G} is selected from S and O, and is preferably S;
X^{2G} is selected from an amide bond, an ether bond, a thioether bond, an ester bond, a thioester bond, a urea bridge, and an amine bond, and is preferably an amide bond.

In line with the above, it is more preferred that R^{G} is selected from the groups of formula (IIc) and (IId): 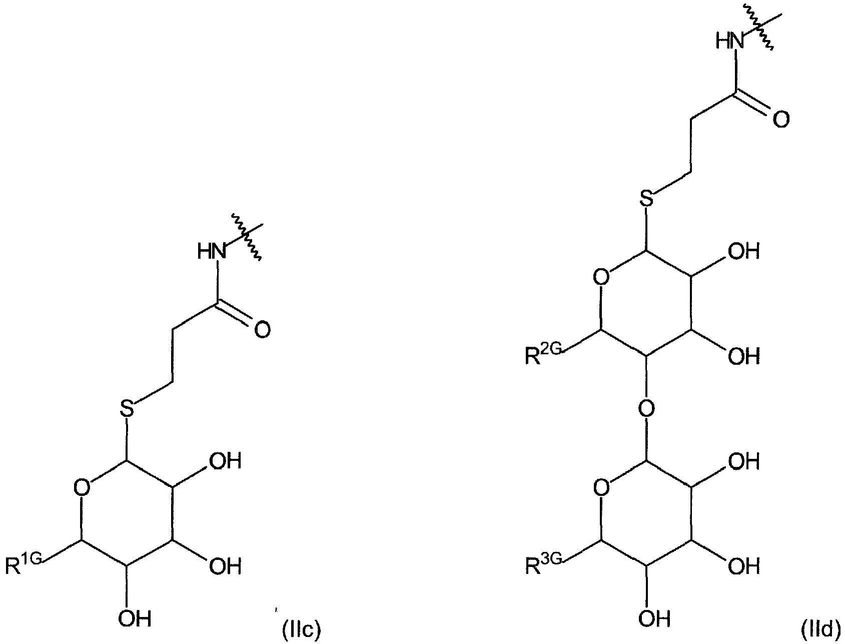 wherein
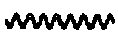 marks the bond which attaches R^{G} to X²; and
R^{1G} is selected from -OH and -CH₂-OH;
R^{2G} is selected from -OH and -CH₂-OH, and is preferably -CH₂-OH; and
R^{3G} is selected from -OH and -CH₂-OH, and is preferably -CH₂-OH.

It is still further preferred that R^{G} is selected from the groups of formula (IIe) to (IIh): 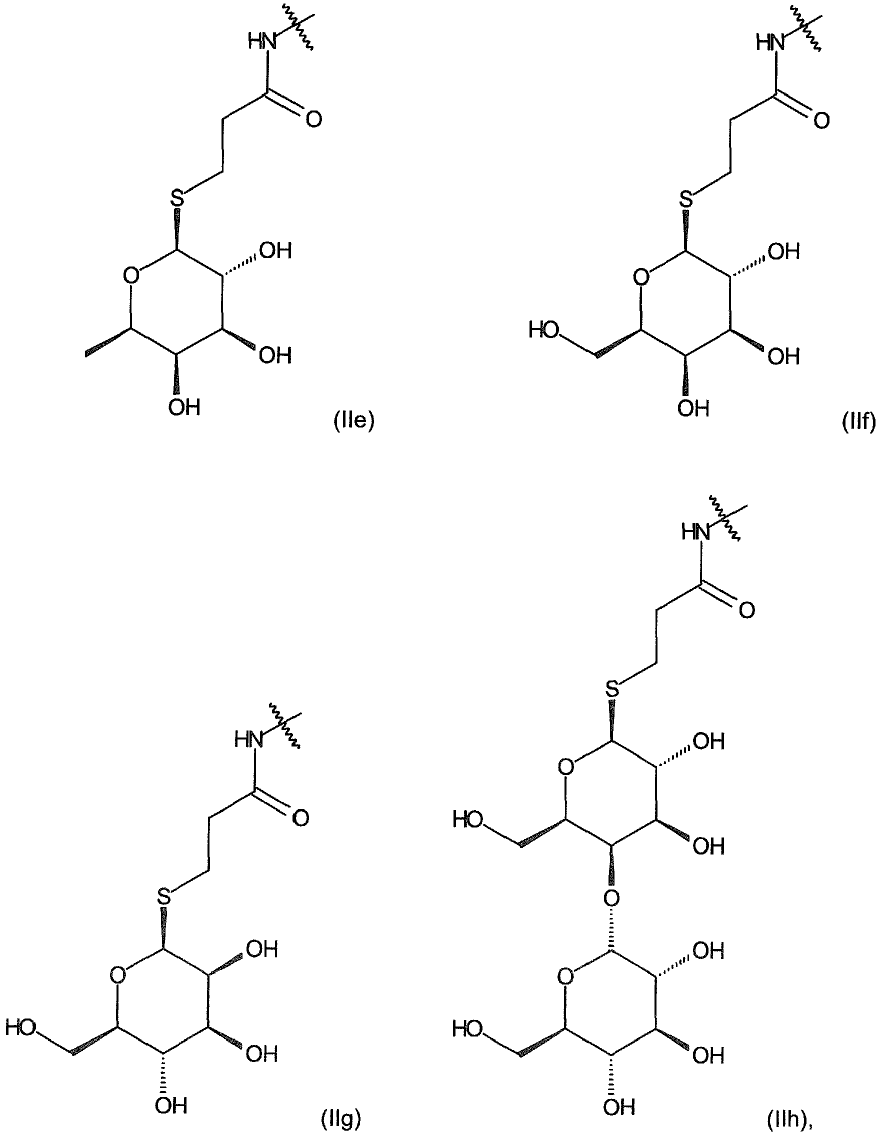 wherein 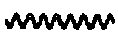 marks the bond which attaches R^{G} to X².

X² in formula (I) is a trivalent coupling group carrying the substituent R^{G}. As will be understood by the skilled reader, the term "coupling group" reflects in particular the fact that the group X² functions to couple (i.e. to covalently bind) the substituent R^{G} to the remainder of the compound of formula (I) or its salt.

Preferably, 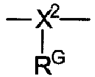 in formula (I) is a group of formula 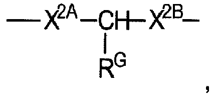 wherein
- X^{2A}: is absent or is selected from an amide bond, an ether bond, a thioether bond, an ester bond, a thioester bond, an urea bridge, and an amine bond, and is preferably an amide bond;
- X^{2B}: is absent or is selected from an amide bond, an ether bond, a thioether bond, an ester bond, a thioester bond, an urea bridge, and an amine bond, and is preferably absent or an amide bond; and
- R^{G}: is as defined as above, including its preferred embodiments.

It will be understood that, in the formulae 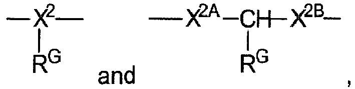 the open bonds representd by "-" indicate bonds attaching the moieties represented by these formulae to their adjacent moieties in formula (I).

In the above preferred formula for -X²(R^{G})-, X^{2A} attaches X² to L², and X^{2B} attaches X² to L¹. It is thus more preferred that 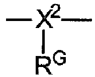 in formula (I) is a group of formula (IIIa) or (IIIb): 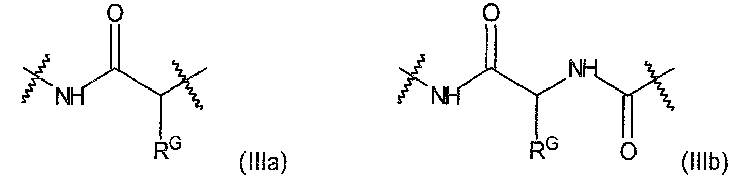 wherein
R^{G} is defined as above, including its preferred embodiments, and wherein the bond marked with 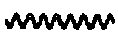 at the N atom attaches X² to L², and the other marked bond attaches X² to L¹.

It is even more preferred that 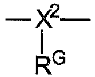 is a group of formula (IIIc) or (IIId), wherein the stereochemistry at the C-atom is indicated to which R^{G} is attached: 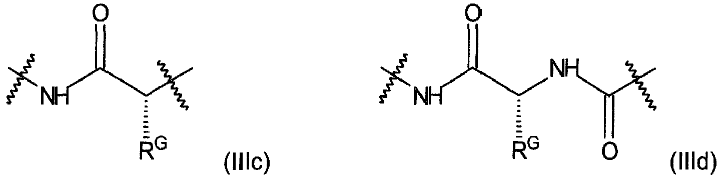 and wherein R^{G} is defined as above, including its preferred embodiments, and wherein the bond marked with 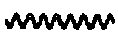 at the N atom attaches X² to L², and the other marked bond attaches X² to L¹.

In line with the above, it will be understood that in particularly preferred compounds of formula (I) and their salts, the moiety -X³-L²-X²(R^{G})-L¹-X¹- in formula (I) is a group of the formula

-C(O)-NH-CH(COOH)-(CH₂)₄-NH-C(O)-CH(R^{G})-(CH₂)₅-C(O)-NH-,

or of the formula

-C(O)-NH-CH(COOH)-(CH₂)₄-NH-C(O)-CH(R^{G})-NH-C(O)-(CH₂)₃-C(O)-NH-,

with the C-terminal being attached to the carbon atom in formula (I) carrying the substituent R², and the N-terminal being attached to the group (CH₂)ₙ in formula (I), and with R^{G} being defined as above, including its preferred embodiments.

In formula (I), a is 0 or 1, and is preferably 1. b is 0 or 1, and is preferably 1. Thus, it is also preferred that a and b are both 1.

R² is an optionally substituted aryl group or an optionally substituted aralkyl group, preferably an optionally substituted aralkyl group. As will be understood, the term "aralkyl group" as used herein refers to an alkyl group wherein a hydrogen atom is replaced by an aryl group as a substituent. Preferably, the aralkyl group is a group wherein one aryl group is bound to an alkanediyl group. The aryl group or aralkyl group represented by R² may be substituted on its aromatic ring with a substituent selected from halogen, preferably I, and -OH. The aryl and the aryl portion of the aralkyl group are preferably selected from phenyl and naphthyl, such as 2-naphthyl. It is more preferred that the aryl and the aryl portion of the aralkyl are phenyl. The alkanediyl portion of the aralkyl group is preferably a C1-C4 alkanediyl group, more preferably a -CH₂- group. Thus, R² is more preferably selected from optionally substituted -CH₂-phenyl and optionally substituted -CH₂-naphthyl, and is most preferably optionally substituted -CH₂-phenyl.

The optionally substituted aryl group and the aryl portion of the optionally substituted aralkyl group, including their preferred embodiments, may be substituted with a substituent selected from halogen, preferably I, and -OH. It will be also be understood that this includes the possibility that one or more than one, e.g. 2 or 3, substituent(s) selected from halogen, preferably I, and -OH is (are) present. However, it is preferred that R² is non-substituted. Thus, R² is most preferably non-substituted -CH₂-phenyl.

R³ is an optionally substituted aryl group or an optionally substituted aralkyl group, preferably an optionally substituted aralkyl group. The aryl group or aralkyl group may be substituted on its aromatic ring with a substituent selected from halogen, preferably I, and -OH. The aryl and the aryl portion of the aralkyl group are preferably selected from phenyl and naphthyl, such as 2-naphthyl. It is more preferred that the aryl and the aryl portion of the aralkyl are phenyl. The alkanediyl portion of the aralkyl group is preferably a C1-C4 alkanediyl group, more preferably a -CH₂- group. Thus, R³ is more preferably optionally substituted -CH₂-phenyl.

The optionally substituted aryl group and the aryl portion of the optionally substituted aralkyl group, including their preferred embodiments, may be substituted with a substituent selected from halogen, preferably I, and -OH. It will be also be understood that this includes the possibility that one or more than one, e.g. 2 or 3, substituent(s) selected from halogen, preferably I, and -OH is (are) present. Preferably, R³ is substituted with one substituent which is -OH, or with a combination of one substituent -OH and one substituent -I. Thus, it is more preferred that R³ is -CH₂-phenyl substituted with one substituent that is -OH, or with a combination of one substituent -OH and one substituent -I. Most preferably, the substituent -OH is present in the para-position of the phenyl ring relative to the -CH₂- group.

In line with the above, it is further preferred for the compounds of formula (I) and their salts that R² is a group of the formula 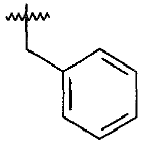 and R³ is a group of the formula 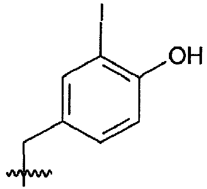 wherein 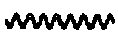 marks the bond which attaches R² and R³, respectively, to the remainder of the compound.

It is even further preferred that R² is a group of the formula 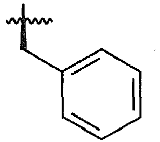 and R³ is a group of the formula 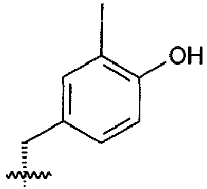 wherein 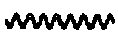 marks the bond which attaches R² and R³, respectively, to the remainder of the compound.

X⁴ is selected from an amide bond, an ether bond, a thioether bond, an ester bond, a thioester bond, a urea bridge, an amine bond, a group of the formula 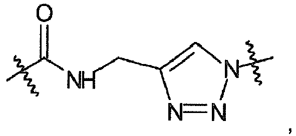 wherein the marked bond at the carbonyl group attaches X⁴ to R^{M} and the other marked bond attaches X⁴ to the remainder of the molecule, and a group of the formula 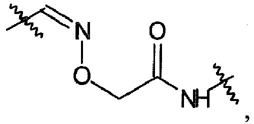 wherein the marked bond at the imino group attaches X⁴ to R^{M} and the other marked bond attaches X⁴ to the remainder of the molecule.
Preferably, X⁴ is selected from an amide bond, a group of the formula 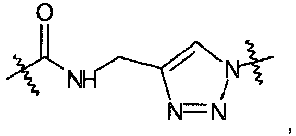 wherein the marked bond at the carbonyl group attaches X⁴ to R^{M} and the other marked bond attaches X⁴ to the remainder of the molecule, and a group of the formula 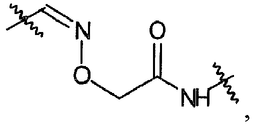 wherein the marked bond at the imino group attaches X⁴ to R^{M} and the other marked bond attaches X⁴ to the remainder of the molecule. In a more preferred embodiment, X⁴ is an amide bond -C(O)-NH- with the carbon atom attached to R^{M}.

R^{M} is a marker group which comprises at least one of
(i) a chelating group optionally containing a chelated non-radioactive or radioactive cation and
(ii) a radiotracer group containing a covalently bound fluorine atom which can be labeled with ¹⁸F by isotopic exchange of ¹⁹F by ¹⁸F or which is labeled with ¹⁸F.

Typically, R^{M} comprises either one of (i) or (ii), and it is not necessary that (i) and (ii) are both comprised in one compound or salt of formula (I) at the same time. It is preferred that R^{M} comprises a chelating group optionally containing a chelated non-radioactive or radioactive cation.

As will be understood by the skilled reader, the above definition according to which R^{M} comprises at least one of the chelating group (i) and the radiotracer group (ii) encompasses the case that R^{M} is a chelating group (i) or a radiotracer group (ii); in this case the chelating group group (i) or radiotracer group (ii) is typically directly bound to X⁴ (if a is 1), to -C(O)-NH-CH(R²)- (if a is 0 and b is 1) or to X³ (if a and b are 0),
and the case that R^{M} comprises, together with the chelating group (i) or a radiotracer group (ii), e.g. a further linker moiety; in this case the chelating group (i) or radiotracer group (ii)can be indirectly bound via this further linker moiety to X⁴ (if a is 1), to -C(O)-NH-CH(R²)- (if a is 0 and b is 1) or to X³ (if a and b are 0).

Incidentally, in the case where R^{M} comprises a chelating group optionally containing a chelated non-radioactive or radioactive cation, it is preferred that X⁴ is an amide bond -C(O)-NH- with the carbon atom attached to R^{M}, or a group of the formula 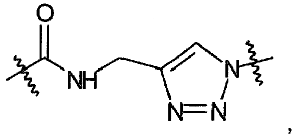 wherein the marked bond at the carbonyl group attaches X⁴ to R^{M} and the other marked bond attaches X⁴ to the remainder of the molecule, and it is more preferred that X⁴ is the amide bond -C(O)-NH- with the carbon atom attached to R^{M}.

The chelating group provided by R^{M} is suitable to form a chelate with a radioactive or non-radioactive cation. Suitable chelating groups for diverse cations are well known in the art, and can be used in the context of the present invention.

The chelating group optionally containing a chelated non-radioactive or radioactive cation is preferably selected from a chelating group comprising at least one of
(i) a macrocyclic ring structure with 8 to 20 ring atoms of which 2 or more, preferably 3 or more, are selected from oxygen atoms, sulfur atoms and nitrogen atoms; and
(ii) an acyclic, open chain chelating structure with 8 to 20 main chain atoms of which 2 or more, preferably 3 or more are heteroatoms selected from oxygen atoms, sulfur atoms and nitrogen atoms.

An exemplary chelating group, and thus also an exemplary group R^{M}, is a residue of a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicyclo[6.6.2]hexadecan (DO2A) 1,4,7,10-tetraazacyclododecan-N,N',N",N'''-tetraacetic acid (DOTA), 2-[1,4,7,10-tetraazacyclododecane-4,7,10-triacetic acid]-pentanedioic acid (DOTAGA), N,N'-dipyridoxylethylendiamine-N,N'-diacetate-5,5'-bis(phosphat) (DPDP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 6-hydrazinyl-N-methylpyridine-3-carboxamide (HYNIC), 1,4,7-triazacyclononan-1-succinic acid-4,7-diacetic acid (NODASA), 1-(1-carboxy-3-carboxypropyl)-4,7-(carbooxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), terpyridin-bis(methyleneamintetraacetic acid (TMT), 1,4,7,10-tetraazacyclotridecan-N,N',N",N'''-tetraacetic acid (TRITA), triethylenetetraaminehexaacetic acid (TTHA), *N,N*'-bis[(6-carboxy-2-pyridyl)methyl]-4,13-diaza-18-crown-6 (H₂macropa), and 4-amino-4-{2-[(3-hydroxy-1,6-dimethyl-4-oxo-1,4-dihydro-pyridin-2-ylmethyl)-carbamoyl]-ethyl} heptanedioic acid bis-[(3-hydroxy-1,6-dimethyl-4-oxo-1,4-dihydro-pyridin-2-ylmethyl)-amide] (THP); which residue is provided by covalently binding a carboxyl group contained in the chelating agent to the remainder of the compound via an ester or an amide bond, preferably an amide bond. It will be understood by the skilled reader that, in formula (I), this ester or amide bond can in this case be represented by or encompassed by X⁴ (if a is 1), or by X³ (if a and b are 0), or the amide bond can be represented by the -C(O)-NH- part of -C(O)-NH-CH(R²)- (if a is 0 and b is 1).

Among these chelating agents, DOTA and DOTAGA are preferred.

It is also preferred that a is 1, and X⁴ is the amide bond -C(O)-NH- with the carbon atom attached to R^{M}.

Thus, it is also preferred that R^{M}-X⁴- is a group of the formula 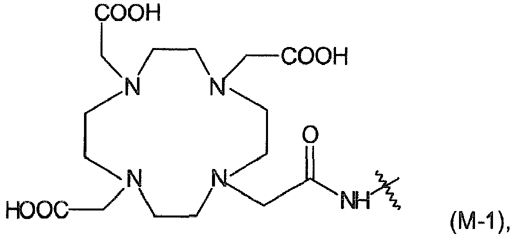 or 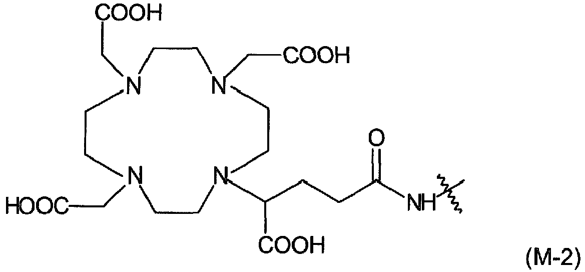 wherein the bond marked with 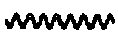 is attached to the remainder of the compound of formula (I), and wherein the chelating group may contain a chelated non-radioactive or radioactive cation.

Exemplary radioactive cations that are optionally chelated by the chelating group are selected from the cations of ⁴⁴Sc, ⁴⁷Sc, ⁵¹Cr, ^{52m}Mn, ⁵⁸Co, ⁵²Fe, ⁵⁶Ni, ⁵⁷Ni, ⁶²Cu, ⁶⁴Cu, ⁶⁷Cu, ⁶⁶Ga, ⁶⁸Ga, ⁶⁷Ga, ⁸⁹Zr, ⁹⁰Y, ⁸⁹Y, ^{94m}Tc, ^{99m}Tc, ⁹⁷Ru, ¹⁰⁵Rh, ¹⁰⁹Pd, ¹¹¹Ag, ^{110m}In, ¹¹¹In, ^{113m}In, ^{114m}In, ^{117m}Sn, ¹²¹Sn, ¹²⁷Te, ¹⁴²Pr, ¹⁴³Pr, ¹⁴⁹Pm, ¹⁵¹Pm, ¹⁴⁹Tb, ¹⁵³Sm, ¹⁵⁷Gd, ¹⁶¹Tb, ¹⁶⁶Ho, ¹⁶⁵Dy, ¹⁶⁹Er, ¹⁶⁹Yb, ¹⁷⁵Yb, ¹⁷²Tm, ¹⁷⁷Lu, ¹⁸⁶Re, ¹⁸⁸Re, ¹⁹¹Pt, ¹⁹⁷Hg, ¹⁹⁸Au, ¹⁹⁹Au, ²¹²Pb, ²⁰³Pb, ²¹¹At, ²¹²Bi, ²¹³Bi, ²²³Ra, ²²⁵Ac, and ²²⁷Th, or a cationic molecule comprising ¹⁸F, such as ¹⁸F-[AlF]²⁺.

More preferred chelated cations are selected from the cations of ⁴⁴Sc, ⁴⁷Sc, ⁶⁴Cu, ⁶⁷Cu, ⁶⁸Ga, ⁹⁰Y, ¹¹¹In, ¹⁶¹Tb, ¹⁶⁶Ho, ¹⁷⁷Lu, ¹⁸⁸Re, ²¹²Pb, ²¹²Bi, ²¹³Bi, ²²⁵Ac, and ²²⁷Th, or a cationic molecule comprising ¹⁸F.

In a further preferred embodiment, X⁴ is a group of the formula 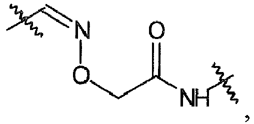 wherein the marked bond at the imino group attaches X⁴ to R^{M} and the other marked bond attaches X⁴ to the remainder of the molecule, and
R^{M} comprises a radiotracer group containing a covalently bound fluorine atom which can be labeled with ¹⁸F by isotopic exchange of ¹⁹F by ¹⁸F or which is labeled with ¹⁸F. A preferred group R^{M} comprising a radiotracer group is a phenyl ring which is substituted by -F, which F is preferably ¹⁸F.

Particularly preferred in the context of the present invention are the compounds of the following formula (Ia) and their pharmaceutically acceptable salts: 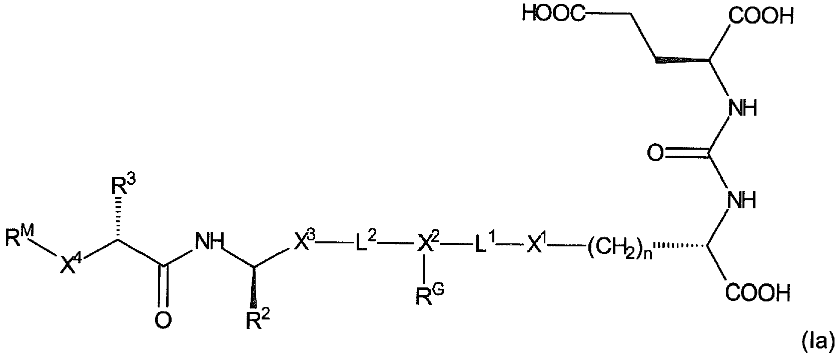 wherein n, X¹, L¹, X², R^{G}, L², X³, R², R³, X⁴ and R^{M} are defined as above, including their preferred meanings.

More preferred in the context of the present invention are the compounds of the following formula (Ib) and their pharmaceutically acceptable salts: 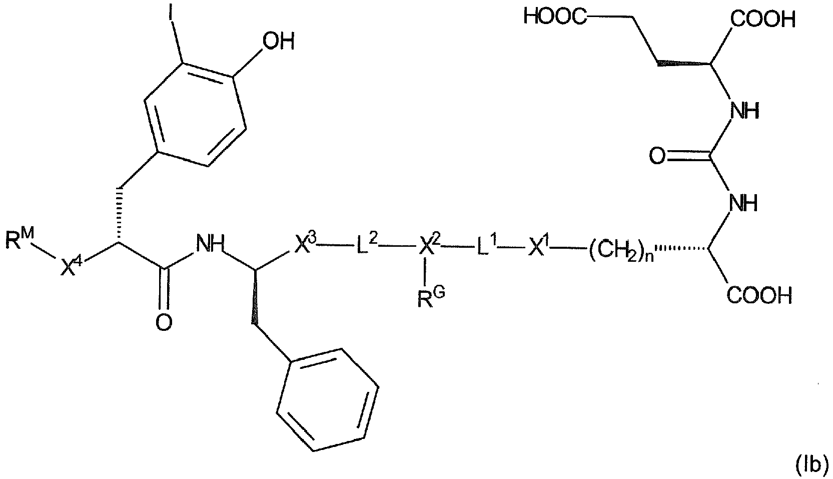 wherein n, X¹, L¹, X², R^{G}, L², X³, X⁴ and R^{M} are defined as above, including their preferred meanings.

Still more preferred in the context of the present invention are the compounds of the following formula (Ic) and their pharmaceutically acceptable salts: 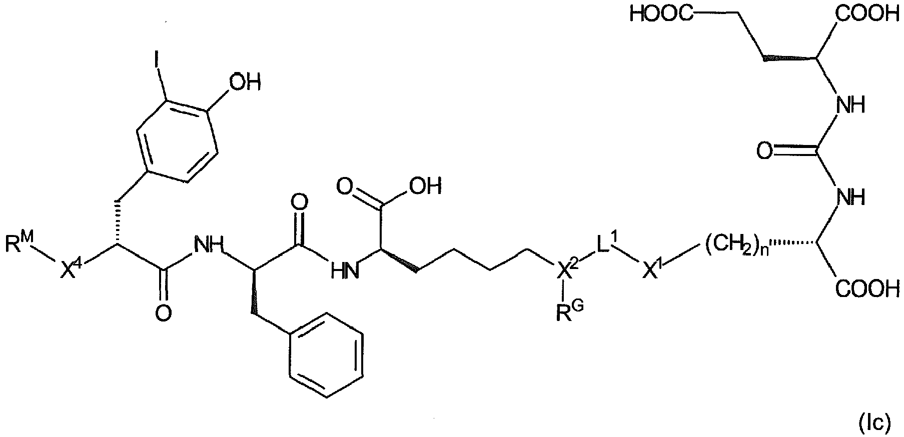 wherein n, X¹, L¹, X², R^{G}, X⁴ and R^{M} are defined as above, including their preferred meanings.

Even more preferred in the context of the present invention are the compounds of the following formula (Id) and their pharmaceutically acceptable salts: 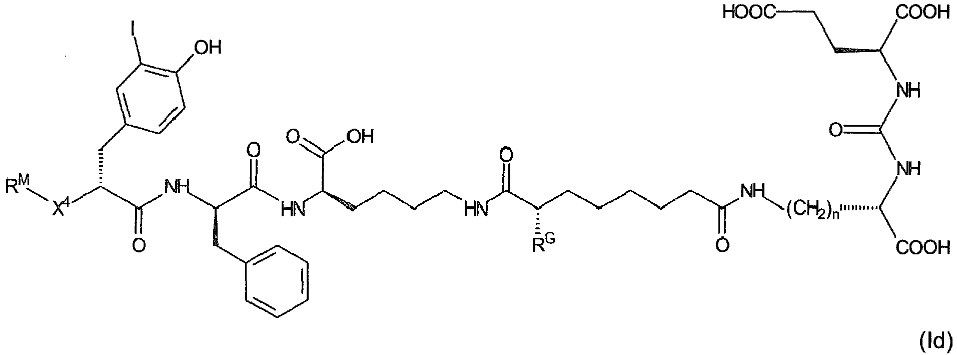 wherein n is 4, and R^{G}, X⁴ and R^{M} are defined as above, including their preferred meanings.

In a further aspect, the present invention relates to a pharmaceutical or diagnostic composition comprising or consisting of one or more compounds or salts in accordance with the present invention.

In a further aspect, the present invention provides a compound or salt in accordance with the invention for use in a method of diagnosing and/or treating
(a) cancer including prostate cancer; or
(b) neoangiogenesis/angiogenesis.

Particularly preferred compounds of the invention are the following: 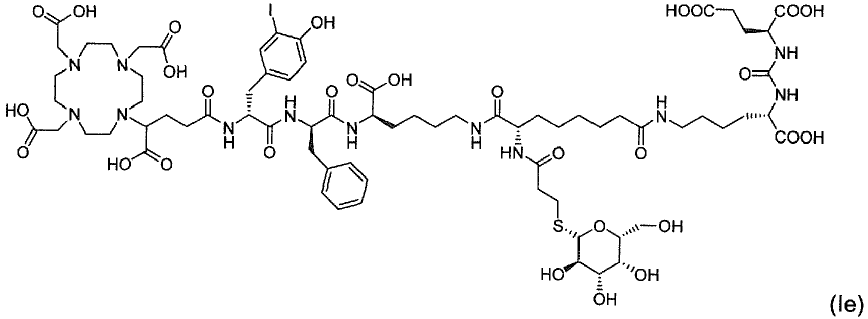 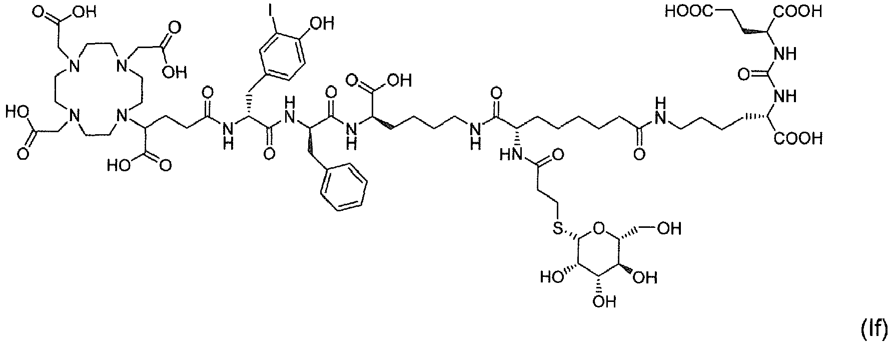 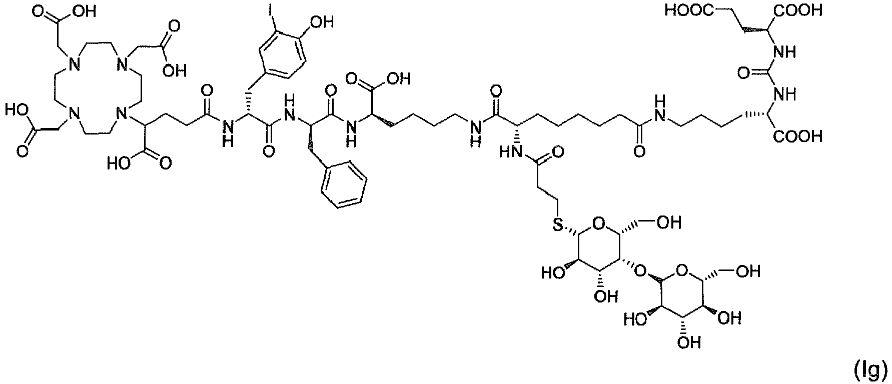 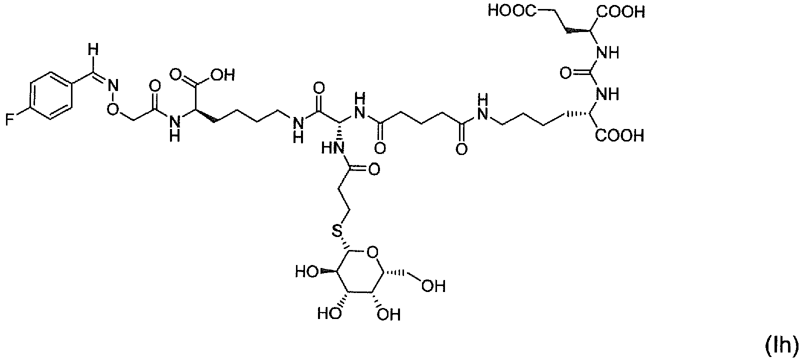 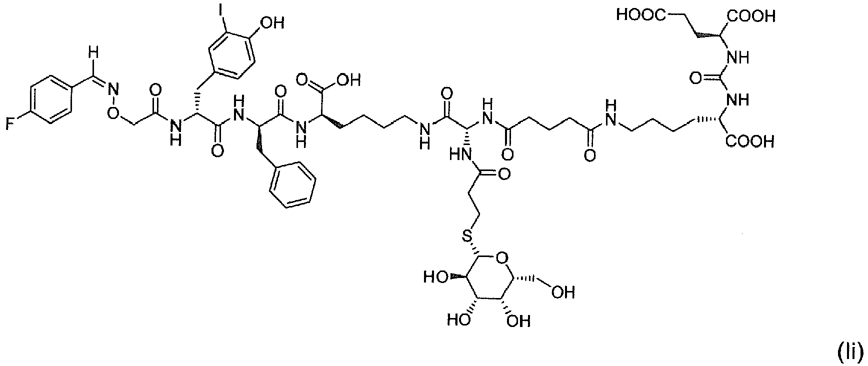 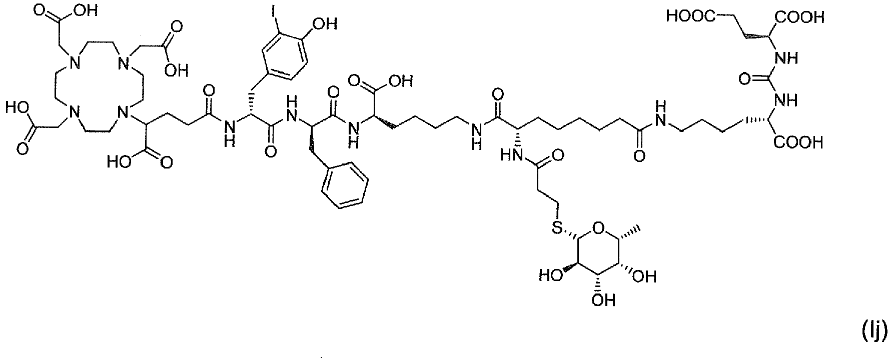 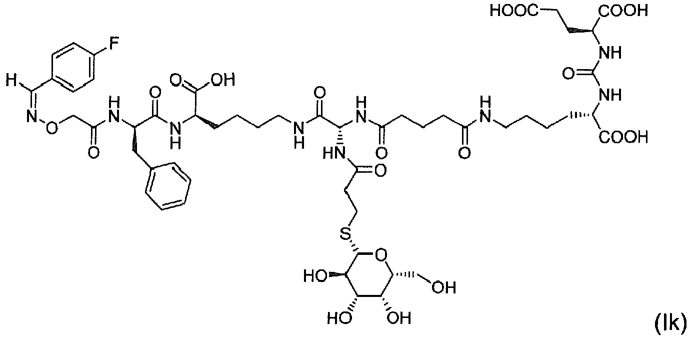 or their pharmaceutically acceptable salts.

Most preferred are the above compounds 13, 14, 15, 23 and 22.

Preferred labeling schemes for these most preferred compounds are as defined herein above.

In a further aspect, the present invention provides a pharmaceutical composition comprising or consisting of one or more compounds or salts of the invention as disclosed herein above.

In a further aspect, the present invention provides a diagnostic composition comprising or consisting of one or more compounds or salts of the invention as disclosed herein above.

In a further aspect, the present invention provides a therapeutic composition comprising or consisting of one or more compounds or salts of the invention as disclosed herein above.

The pharmaceutical composition may further comprise pharmaceutically acceptable carriers, excipients and/or diluents. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the pancreas or into a brain artery or directly into brain tissue. The compositions may also be administered directly to the target site, e.g., by biolistic delivery to an external or internal target site, like the pancreas or brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Pharmaceutically active matter may be present in amounts between 0,1 ng and 10 mg/kg body weight per dose; however, doses below or above this exemplary range are envisioned, especially considering the aforementioned factors.

The above applies *mutatis mutandis* also to diagnostic compositions.

To the extent the above disclosed pharmaceutical composition, diagnostic composition and therapeutic composition comprises one or more compounds of the invention, it is preferred that no further pharmaceutically active compounds, diagnostically active compounds or therapeutically active compounds are present. In the alternative, further therapeutically active, diagnostically active or pharmaceutically active compounds may be present, for example, anticancer agents.

Combination of a therapeutic treatment with the compounds of the present invention might have a synergistic or cumulative treatment effect, similar to the treatment of neuroendocrine tumors with [¹⁷⁷Lu]DOTATATE radiotherapy in combination with chemotherapy or immunotherapies. A first phase 3 study comparing the combination of ¹⁷⁷Lu PRRT and capecitabine (Xeloda; Genentech), an oral chemotherapy agent, with [¹⁷⁷Lu]DOTATATE alone has been started at Erasmus MC, Rotterdam in 2017 (van Essen M, Krenning EP, Kam BL, de Herder WW, van Aken MO, Kwekkeboom DJ. Report on short-term side effects of treatments with 177Lu-octreotate in combination with capecitabine in seven patients with gastroenteropancreatic neuroendocrine tumours. Eur J Nucl Med Mol Imaging. 2008;35:743-748).

Further studies on combination therapies, named peptide receptor chemoradionuclide therapy (PRCRT), have recently been published (Kong G, Callahan J, Hofman MS, et al. High clinical and morphologic response using 90Y-DOTA-octreotate sequenced with 177Lu-DOTA-octreotate induction peptide receptor chemoradionuclide therapy (PRCRT) for bulky neuroendocrine tumours. Eur J Nucl Med Mol Imaging. 2017;44:476-489). Similar "combined treatment approaches" will be carried out in the near future to improve the efficiency of PSMA-targeted radioligand therapies.

In a further aspect, the present invention provides one or more compounds of the invention as disclosed herein above for use in medicine.

Preferred uses in medicine are in nuclear medicine such as nuclear diagnostic imaging, also named nuclear molecular imaging, and/or targeted radiotherapy of diseases associated with an overexpression, preferably of PSMA on the diseased tissue.

In a further aspect, the present invention provides a compound of the invention as defined herein above for use in a method of diagnosing and/or staging cancer, preferably prostate cancer.

Preferred indications are the detection or staging of cancer, such as, but not limited high grade gliomas, lung cancer and especially prostate cancer and metastasized prostate cancer, the detection of metastatic disease in patients with primary prostate cancer of intermediate-risk to high-risk, and the detection of metastatic sites, even at low serum PSA values in patients with biochemically recurrent prostate cancer. Another preferred indication is the imaging and visualization of neoangiogensis.

In terms of medical indications to be subjected to therapy, especially radiotherapy, cancer is a preferred indication. Prostate cancer is a particularly preferred indication.

In a further aspect, the present invention provides a compound of the invention as defined herein above for use in a method of diagnosing and/or staging cancer, preferably prostate cancer.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

In particular, the invention includes the following items:
1. A compound of formula (I), or a pharmaceutically acceptable salt thereof, 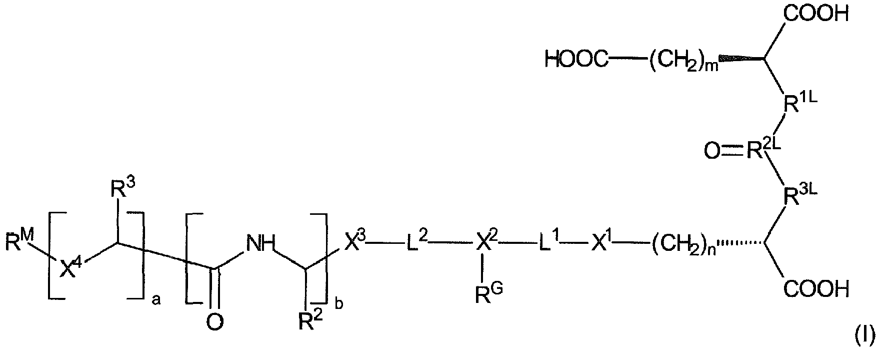 wherein:
   - m: is an integer of 2 to 6, preferably 2 to 4, more preferably 2;
   - n: is an integer of 2 to 6, preferably 2 to 4, more preferably 4;
   - R^{1L}: is CH₂, NH or O, preferably NH;
   - R^{2L}: is C or P(OH), preferably C;
   - R^{3L}: is CH₂, NH or O, preferably NH;
   - X¹: is selected from an amide bond, an ether bond, a thioether bond, an ester bond, a thioester bond, a urea bridge, and an amine bond; and is preferably an amide bond;
   - L¹: is an alkanediyl group, preferably a C2-C10 alkanediyl group, more preferably a C3-C6 alkanediyl group, and most preferably a C3 or C5 alkanediyl group, which alkanediyl group is optionally substituted by a group selected from-COOH and -NH₂;
   - X²: is a trivalent coupling group carrying the substituent R^{G};
   - L²: is an alkanediyl group, preferably a C2-C10 alkanediyl group, more preferably a C3-C6 alkanediyl group, and most preferably a C5 alkanediyl group, said alkanediyl group being optionally substituted by a group selected from -COOH and -NH₂;
   - X³: is selected from an amide bond, an ether bond, a thioether bond, an ester bond, a thioester bond, a urea bridge, and an amine bond, and is preferably an amide bond;
   - a: is 0 or 1, preferably 1;
   - b: is 0 or 1, preferably 1;
   - R²: is an optionally substituted aryl group or an optionally substituted aralkyl group, which aryl group or aralkyl group may be substituted on its aromatic ring with a substituent selected from halogen, preferably I, and -OH;
   - R³: is an optionally substituted aryl group or an optionally substituted aralkyl group, which aryl group or aralkyl group may be substituted on its aromatic ring with a substituent selected from halogen, preferably I, and -OH;
   - X⁴: is selected from an amide bond, an ether bond, a thioether bond, an ester bond, a thioester bond, a urea bridge, an amine bond, a group of the formula 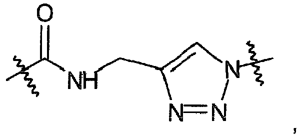 wherein the marked bond at the carbonyl group attaches X⁴ to R^{M} and the other marked bond attaches X⁴ to the remainder of the molecule, and a group of the formula 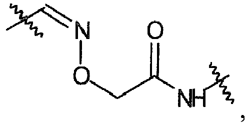 wherein the marked bond at the imino group attaches X⁴ to R^{M} and the other marked bond attaches X⁴ to the remainder of the molecule;
   and is preferably an amide bond;
   - R^{M}: is a marker group which comprises at least one of
   (i) a chelating group optionally containing a chelated non-radioactive or radioactive cation and
   (ii) a radiotracer group containing a covalently bound fluorine atom which can be labeled with ¹⁸F by isotopic exchange of ¹⁹F by ¹⁸F or which is labeled with ¹⁸F; and
   - R^{G}: is a group of the formula (IIa) or (IIb): 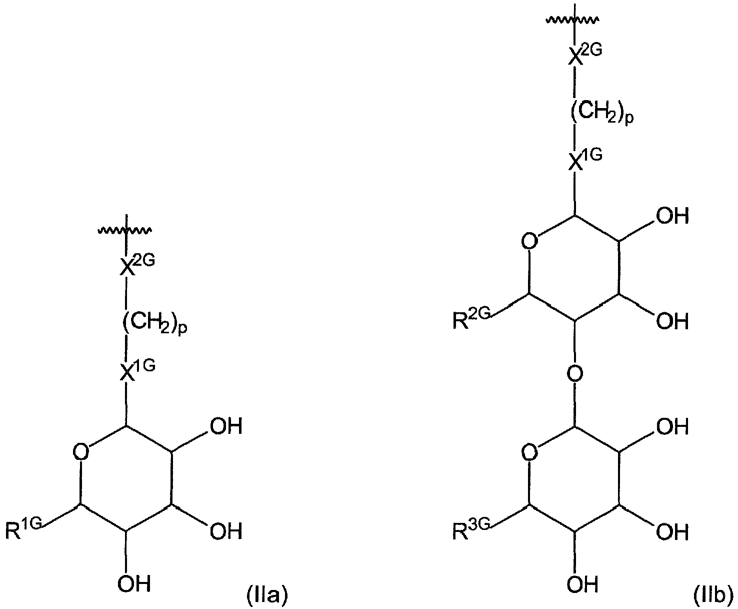
   wherein
   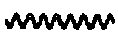 marks the bond which attaches R^{G} to X²;
   p is an integer of 1 to 5, preferably 2 or 3, and more preferably 2;
   R^{1G} is selected from -OH and -CH₂-OH;
   R^{2G} is selected from -OH and -CH₂-OH, and is preferably -CH₂-OH;
   R^{3G} is selected from -OH and -CH₂-OH, and is preferably -CH₂-OH;
   X^{1G} is selected from S and O, and is preferably S;
   X^{2G} is selected from an amide bond, an ether bond, a thioether bond, an ester bond, a thioester bond, an urea bridge, and an amine bond, and is preferably an amide bond.
2. The compound or salt of item 1, wherein m is 2, n is 2 or 4, R^{1L} is NH, R^{2L} is C, and R^{3L} is NH.
3. The compound or salt of item 1 or 2, wherein n is 2 or 4.
4. The compound or salt of any one of items 1 to 3, wherein L¹ is a linking group of the formula -(CH₂)₅-.
5. The compound or salt of any of items 1 to 4, wherein the alkanediyl group L² carries one substituent which is a -COOH group.
6. The compound or salt of item 5, wherein the alkanediyl group L² carries one substituent which is a -COOH group and which is present on the carbon atom attached to X³.
7. The compound or salt of item 6, wherein the alkanediyl group L² is a group of the formula -CH(COOH)-(CH₂)₄-, with the carbon atom carrying the substitutent -COOH being attached to X³.
8. The compound or salt of any of items 1 to 7, wherein X¹ and X³ are both amide bonds.
9. The compound or salt of any of items 1 to 8, wherein X¹ is an amide bond -C(O)-NH-
   (a) with the nitrogen atom attached to the group (CH₂)ₙ, wherein n is 4; or
   (b) with the carbon atom attached to the group (CH₂)ₙ, wherein n is 2; and
   X³ is an amide bond -C(O)-NH- with the nitrogen atom attached to L².
10. The compound or salt of any of items 1 to 9, wherein 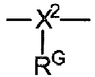 is a group of formula 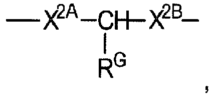 wherein
   - X^{2A}: is absent or is selected from an amide bond, an ether bond, a thioether bond, an ester bond, a thioester bond, an urea bridge, and an amine bond, and is preferably an amide bond;
   - X^{2B}: is absent or is selected from an amide bond, an ether bond, a thioether bond, an ester bond, a thioester bond, an urea bridge, and an amine bond, and is preferably absent or an amide bond;
   - R^{G}: is as defined in the preceding items;
   and wherein X^{2A} attaches X² to L², and X^{2B} attaches X² to L¹.
11. The compound or salt of item 10, wherein 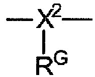 is a group of formula (IIIa) or (IIIb): 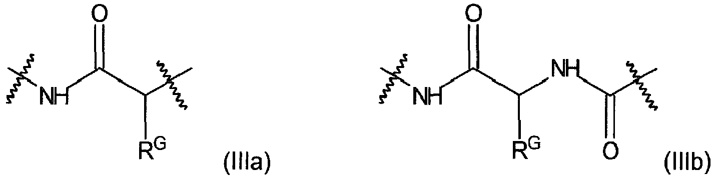 wherein
   - R^{G}: is as defined in the preceding items;
   and wherein the bond marked with 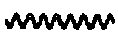 at the N atom attaches X² to L², and the other marked bond attaches X² to L¹.
12. The compound or salt of any of items 1 to 11, wherein -X³-L²-X²(R^{G})-L¹-X¹- is a group of the formula

   -C(O)-NH-CH(COOH)-(CH₂)₄-NH-C(O)-CH(R^{G})-(CH₂)₅-C(O)-NH-

   or of the formula

   -C(O)-NH-CH(COOH)-(CH₂)₄-NH-C(O)-CH(R^{G})-NH-C(O)-(CH₂)₃-C(O)-NH-,

   with the C-terminal in each formula being attached to the carbon atom in formula (I) carrying the substituent R², and the N-terminal in each formula being attached to the group (CH₂)ₙ in formula (I), and with R^{G} being defined as in the preceding items.
13. The compound or salt of item 11, wherein 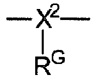 is a group of formula (IIIc) or (IIId): 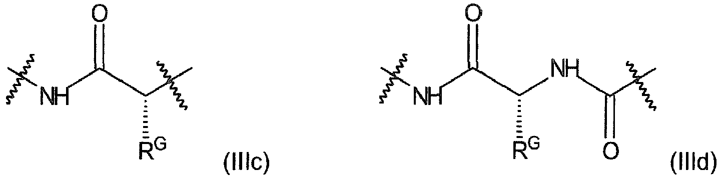 wherein
   - R^{G}: is as defined in the preceding items;
   and wherein the bond marked with 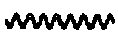 at the N atom attaches X² to L², and the other marked bond attaches X² to L¹.
14. The compound or salt of any of items 1 to 13, wherein R^{G} is selected from the groups of formula (IIc) and (IId): 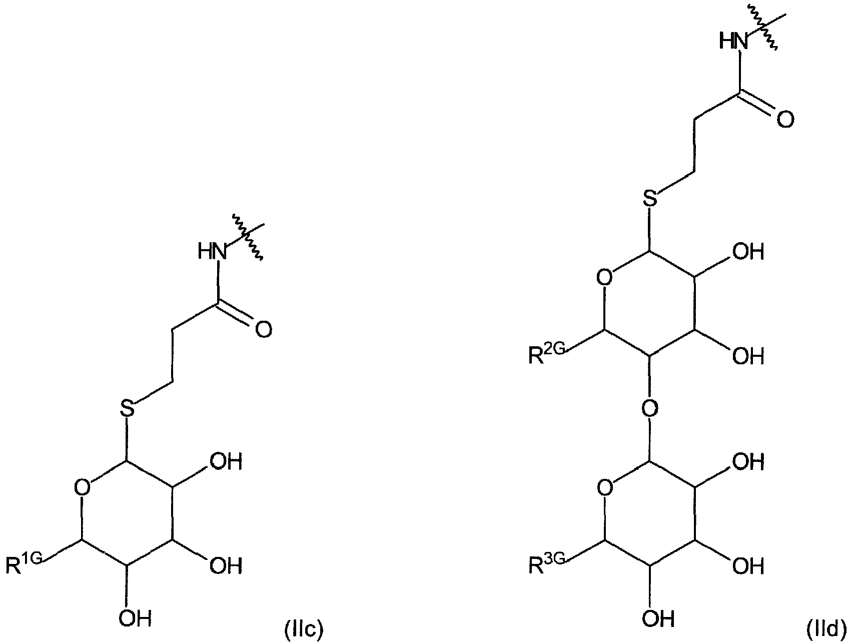 wherein
   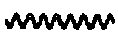 marks the bond which attaches R^{G} to X²; and
   R^{1G} is selected from -OH and -CH₂-OH;
   R^{2G} is selected from -OH and -CH₂-OH, and is preferably -CH₂-OH; and
   R^{3G} is selected from -OH and -CH₂-OH, and is preferably -CH₂-OH.
15. The compound or salt of item 14, wherein R^{G} is selected from the groups of formula (IIe) to (IIh): 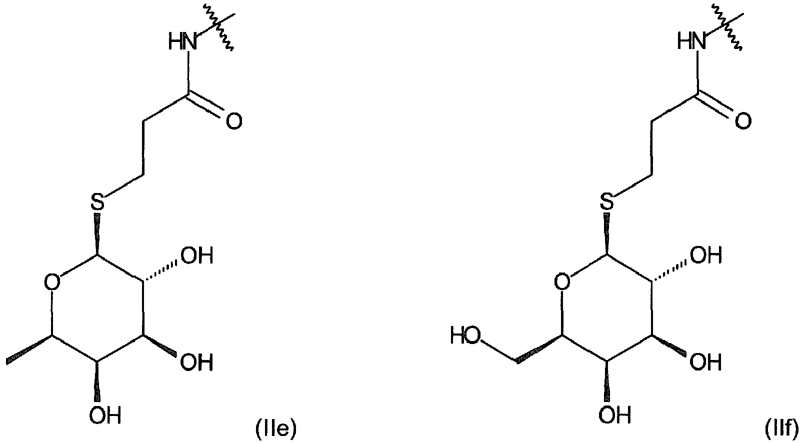 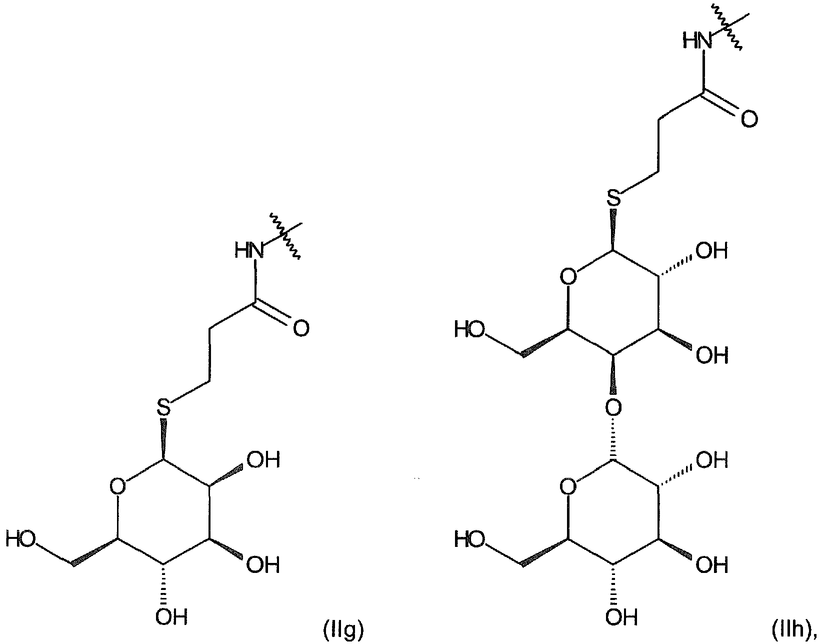 wherein 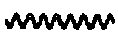 marks the bond which attaches R^{G} to X².
16. The compound or salt of any of items 1 to 15, wherein a is 1 and b is 1.
17. The compound or salt of any of items 1 to 16, wherein R² is an aralkyl group of the formula -CH₂-phenyl or -CH₂-naphthyl, wherein the phenyl and the naphtyl group are optionally substituted with a substituent selected from halogen, preferably I, and -OH.
18. The compound or salt of any of items 1 to 16, wherein R² is an aralkyl group of the formula -CH₂-phenyl.
19. The compound or salt of any of items 1 to 18, wherein R³ is an aralkyl group of the formula -CH₂-phenyl or -CH₂-naphthyl, wherein the phenyl and the naphtyl group are optionally substituted with a substituent selected from halogen, preferably I, and -OH.
20. The compound or salt of any of items 1 to 18, wherein R³ is an aralkyl group of the formula -CH₂-phenyl, wherein the phenyl group is substituted with at least one substituent selected from -I and -OH, and is preferably substituted with -I and -OH.
21. The compound or salt of any of items 1 to 18, wherein R² is a group of the formula 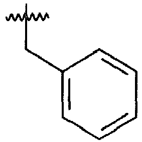 and R³ is a group of the formula 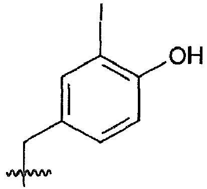 wherein 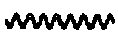 marks the bond which attaches R² and R³, respectively, to the remainder of the molecule.
22. The compound or salt of any of items 1 to 21, wherein X⁴ is an amide bond or a group of the formula 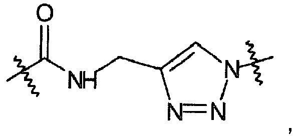 wherein the marked bond at the carbonyl group attaches X⁴ to R^{M} and the other marked bond attaches X⁴ to the remainder of the molecule.
23. The compound or salt of any of items 1 to 21, wherein X⁴ is an amide bond -C(O)-NH-with the carbon atom attached to R^{M}.
24. The compound or salt of any of items 1 to 23, wherein R^{M} comprises a chelating group optionally containing a chelated non-radioactive or radioactive cation or is a chelating group optionally containing a chelated non-radioactive or radioactive cation.
25. The compound or salt of any one of items 1 to 24, wherein the chelating group comprises at least one of
   (i) a macrocyclic ring structure with 8 to 20 ring atoms of which 2 or more, preferably 3 or more, are selected from oxygen atoms, sulfur atoms and nitrogen atoms; and
   (ii) an acyclic, open chain chelating structure with 8 to 20 main chain atoms of which 2 or more, preferably 3 or more are heteroatoms selected from oxygen atoms, sulfur atoms and nitrogen atoms.
26. The compound or salt of any one of items 1 to 25, wherein the chelating group is a residue of a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicyclo[6.6.2]hexadecan (DO2A) 1,4,7,10-tetraazacyclododecan-N,N',N",N'''-tetraacetic acid (DOTA), 2-[1,4,7,10-tetraazacyclododecane-4,7,10-triacetic acid]-pentanedioic acid (DOTAGA), N,N'-dipyridoxylethylendiamine-N,N'-diacetate-5,5'-bis(phosphat) (DPDP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 6-hydrazinyl-N-methylpyridine-3-carboxamide (HYNIC), 1,4,7-triazacyclononan-1-succinic acid-4,7-diacetic acid (NODASA), 1-(1-carboxy-3-carboxypropyl)-4,7-(carbooxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), terpyridin-bis(methyleneamintetraacetic acid (TMT), 1,4,7,10-tetraazacyclotridecan-N,N',N",N'''-tetraacetic acid (TRITA), and triethylenetetraaminehexaacetic acid (TTHA), *N,N*'-bis[(6-carboxy-2-pyridyl)methyl]-4,13-diaza-18-crown-6 (H₂macropa), and 4-amino-4-{2-[(3-hydroxy-1,6-dimethyl-4-oxo-1,4-dihydro-pyridin-2-ylmethyl)-carbamoyl]-ethyl} heptanedioic acid bis-[(3-hydroxy-1,6-dimethyl-4-oxo-1,4-dihydro-pyridin-2-ylmethyl)-amide] (THP);
   which residue is provided by covalently binding a carboxyl group contained in the chelating agent to the remainder of the compound via an ester or an amide bond.
27. The compound or salt of item 26, wherein the chelating agent is selected from DOTA and DOTAGA.
28. The compound or salt of item 26 or 27, wherein a is 1, and X⁴ is the amide bond attaching the chelating group to the remainder of the molecule.
29. The compound or salt of any one of items 24 to 28, wherein a is 1 and R^{M}-X⁴- is a group of the formula 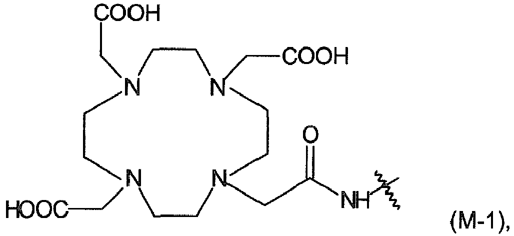 or 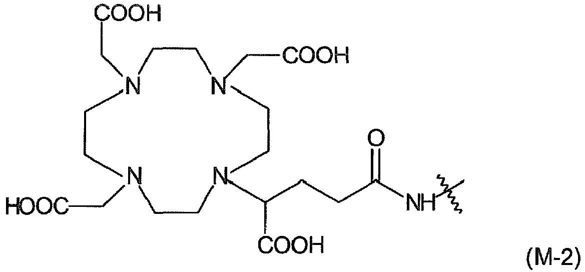 wherein the bond marked with 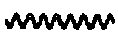 is attached to the remainder of the compound of formula (I), and wherein the chelating group may contain a chelated non-radioactive or radioactive cation.
30. The compound or salt of any of items 24 to 29, wherein the chelating group comprises a chelated cation, preferably a chelated radioactive cation selected from a cation of ⁴⁴Sc, ⁴⁷Sc, ⁵¹Cr, ^{52m}Mn, ⁵⁸Co, ⁵²Fe, ⁵⁶Ni, ⁵⁷Ni, ⁶²Cu, ⁶⁴Cu, ⁶⁷Cu, ⁶⁶Ga, ⁶⁸Ga, ⁶⁷Ga, ⁸⁹Zr, ⁹⁰Y, ⁸⁹Y, ^{94m}Tc, ^{99m}Tc, ⁹⁷Ru, ¹⁰⁵Rh, ¹⁰⁹Pd, ¹¹¹Ag, ^{110m}In, ¹¹¹In, ^{113m}In, ^{114m}In, ^{117m}Sn, ¹²¹Sn, ¹²⁷Te, ¹⁴²Pr, ¹⁴³Pr, ¹⁴⁹Pm, ¹⁵¹Pm, ¹⁴⁹Tb, ¹⁵³Sm, ¹⁵⁷Gd, ¹⁶¹Tb, ¹⁶⁶Ho, ¹⁶⁵Dy, ¹⁶⁹Er, ¹⁶⁹Yb, ¹⁷⁵Yb, ¹⁷²Tm, ¹⁷⁷Lu, ¹⁸⁶Re, ¹⁸⁸Re, ¹⁹¹Pt, ¹⁹⁷Hg, ¹⁹⁸Au, ¹⁹⁹Au, ²¹²Pb, ²⁰³Pb, ²¹¹At., ²¹²Bi, ²¹³Bi, ²²³Ra, ²²⁵Ac, and ²²⁷Th, or a cationic molecule comprising ¹⁸F, such as ¹⁸F-[AlF]²⁺.
31. The compound or salt of item 30, wherein the chelating group comprises a chelated cation selected from a cation of ⁴⁴Sc, ⁴⁷Sc, ⁶⁴Cu, ⁶⁷Cu, ⁶⁸Ga, ⁹⁰Y, ¹¹¹In, ¹⁶¹Tb, ¹⁶⁶Ho, ¹⁷⁷Lu, ¹⁸⁸Re, ²¹²Pb, ²¹²Bi, ²¹³Bi, ²²⁵Ac, and ²²⁷Th or a cationic molecule comprising ¹⁸F.
32. The compound or salt of any of items 1 to 21, wherein X⁴ is a group of the formula 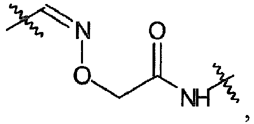 wherein the marked bond at the imino group attaches X⁴ to R^{M} and the other marked bond attaches X⁴ to the remainder of the molecule, and
   R^{M} is a radiotracer group containing a covalently bound fluorine atom which can be labeled with ¹⁸F by isotopic exchange of ¹⁹F by ¹⁸F or which is labeled with ¹⁸F.
33. The compound or salt of item 32, wherein R^{M} is a phenyl ring which is substituted with -F.
34. The compound or salt of any of items 1 to 33, which has the formula (Ia): 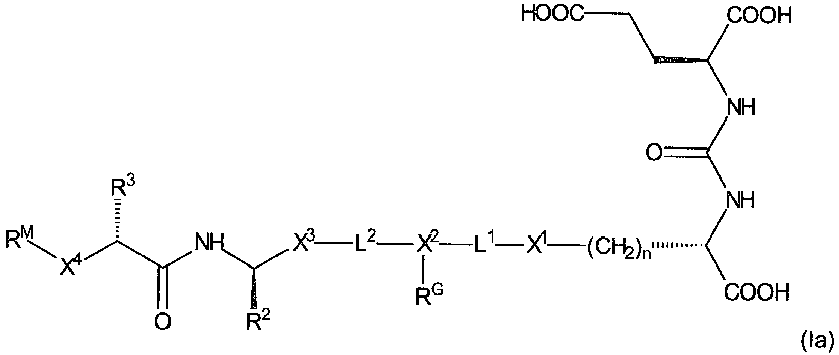 wherein n, X¹, L¹, X², R^{G}, L², X³, R², R³, X⁴ and R^{M} are defined as in the preceding items.
35. The compound or salt of item 34, which has the formula (Ib): 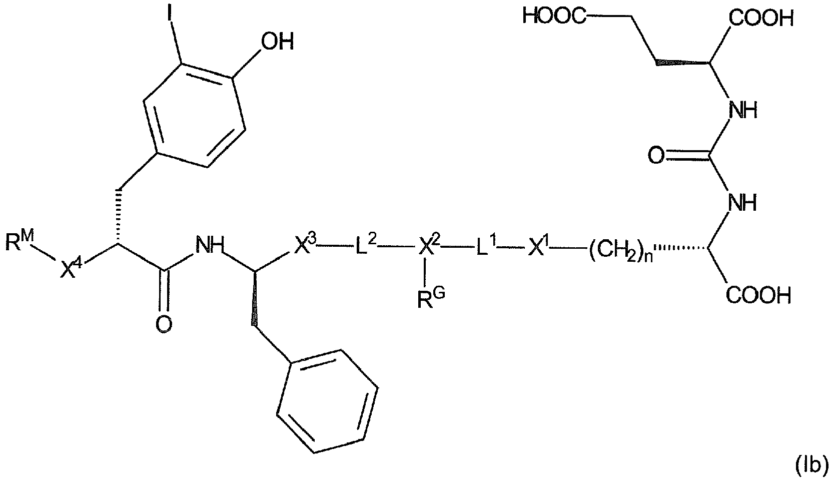 wherein n, X¹, L¹, X², R^{G}, L², X³, X⁴ and R^{M} are defined as in the preceding items.
36. The compound or salt of item 35, which has the formula (Ic): 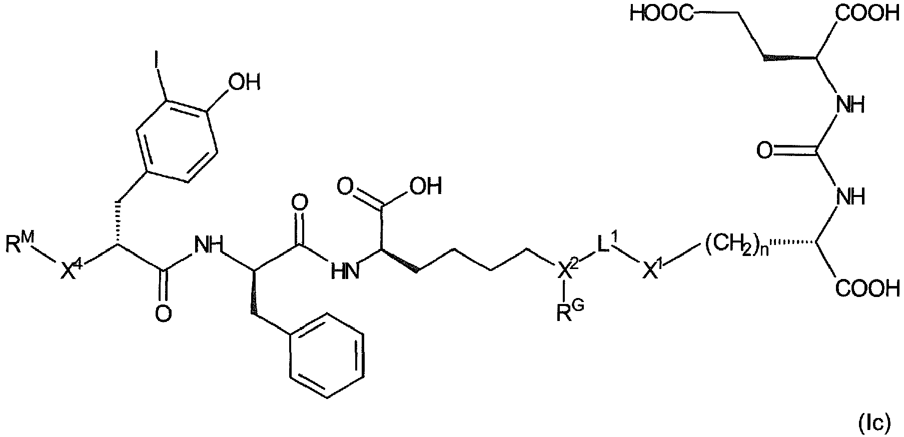 wherein n, X¹, L¹, X², R^{G}, X⁴ and R^{M} are defined as in the preceding items.
37. The compound or salt of item 36, which has the formula (Id): 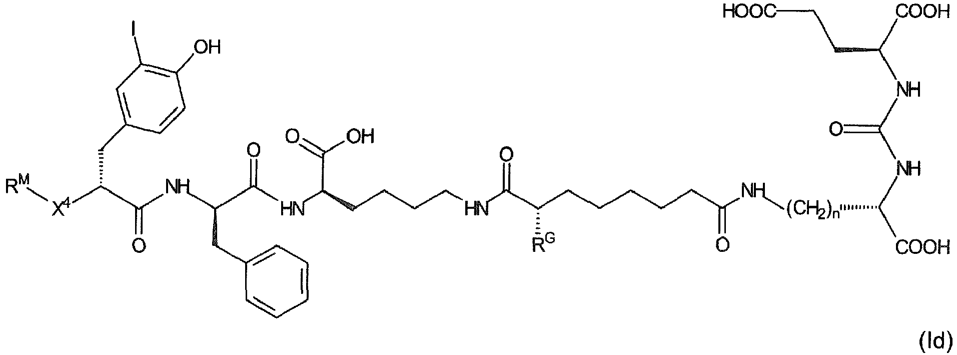 wherein n is 4, and R^{G}, X⁴ and R^{M} are defined as in the preceding items.
38. The compound of any one of the preceding items, wherein said compound is one of the following: 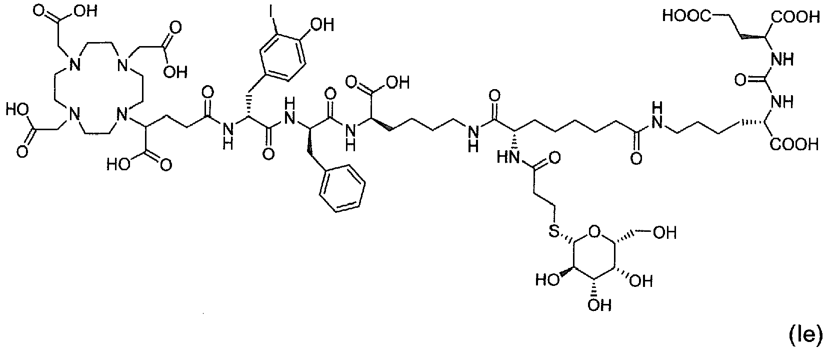 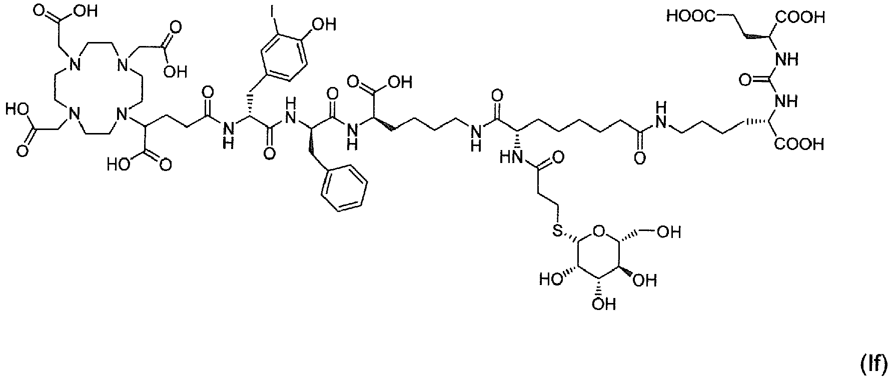 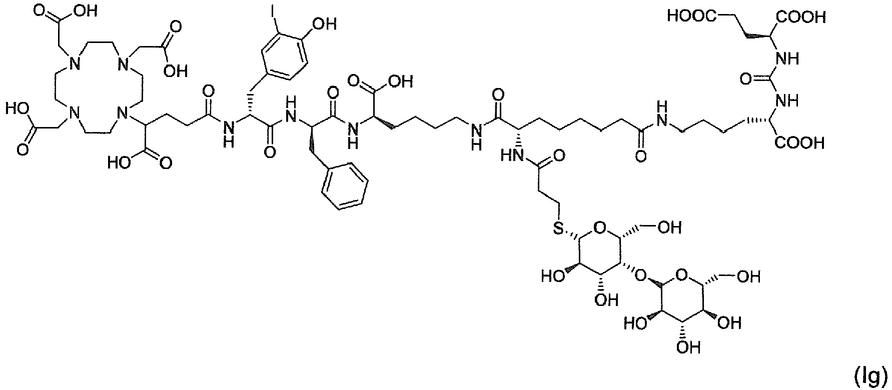 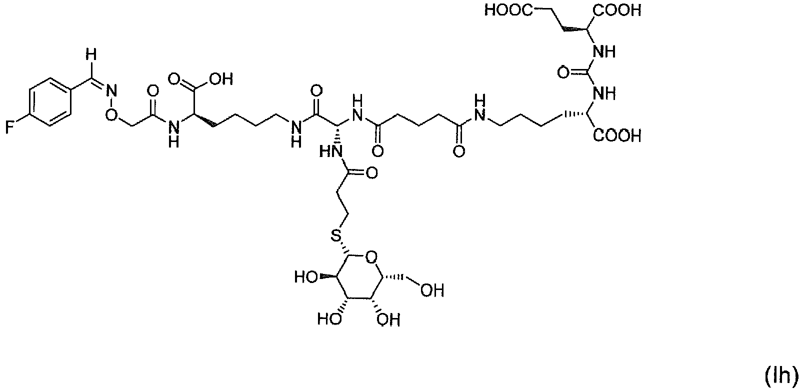 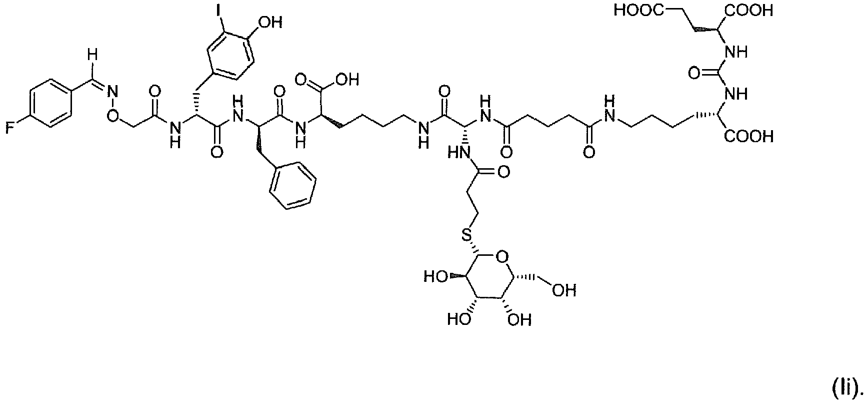
39. A pharmaceutical or diagnostic composition comprising or consisting of one or more compounds or salts in accordance with any one of items 1 to 38.
40. A compound or salt in accordance with any one of items 1 to 38 for use in a method of diagnosing and/or treating
   (a) cancer including prostate cancer; or
   (b) neoangiogenesis/angiogenesis.

The figures show:
**Figure 1****:** Chemical Structure of PSMA I&T
**Figure 2****:** (a) Preferred attachment site for further conjugation with carbohydrates; (b) Thio-linked cyclic carbohydrate derivatives of fucose, galactose, mannose and cellobiose. R in (a) designates fragments of (b) without R, and R in (b) designates the fragments (a) without R.
**Figure 3****: Blood cell binding of ¹⁷⁷Lu-13 and ¹⁷⁷Lu-18.** Samples of 1 mL blood were incubated with appr. 1,0 MBq of the radiolabeled PSMA inhibitors or for blockade with 100 µM PMPA; Blood samples were stepwise centrifuged at 700 rpm for 5 min with two subsequent washing steps (PBS) to separate red blood cells (erythrocytes). The combined supernatant was centrifuged at 6200 rpm for 5 min and again washed twice to obtain the white cell (leukocytes) fraction and separated from the supernatant. Equal volumes of each fraction were measured in a γ-counter.
**Figure 4****: Metabolic stability of ¹⁷⁷Lu-13.** Radio-HPLC analyses of quality control (QC) and extracts from homogenized organs and body fluids from a male CB-17 SCID mouse (60 min **p.i., 25 MBq ¹⁷⁷Lu-13).** Chromolith column, binary gradient, flow rate 3 mL/min, 3% MeCN to 95% MeCN in 6 min, 95% MeCN for 3 min.
**Figure 5****: Maximum intensity projections (MIP) of µPET scans in LNCaP xenograft bearing mice.** Dynamic MIP (summed up frames 1 to 1.5 h p.i.) of **A)** 7 MBq ⁶⁸Ga-**18** (PSMA I&T) and **B)** 12 MBq ⁶⁸Ga-**13** (0.15 to 0.20 nmol peptide, respectively). **C)** Static MIP (1 h p.i. for 15 min) of ⁶⁸Ga-**13** + PMPA coinjection (8 mg/kg).
**Figure 6****: Time-activity-curves (logarithmic and linear plot) in % ID/mL. A) ⁶⁸Ga-14 in selected organs.** Comparison of ⁶⁸Ga-14 vs. ⁶⁸Ga-1 (PSMA I&T) in B) tumor and C) kidney uptake. Data obtained from µPET imaging in CB-17 SCID mice.
**Figure 7****: Biodistribution data (in %ID/g).** Biodistribution in healthy CB-17 SCID mice for ⁶⁸Ga-**18** at 1 h p.i. and in LNCaP tumor xenograft bearing CB-17 SCID mice for ⁶⁸Ga-**13** at 1 h and 3 h p.i. (n = 4; respectively).
**Figure 8****:** Tumor to tissue ratios of ⁶⁸Ga-**18** and ⁶⁸Ga-**13** after 1h p.i. in LNCaP xenograft bearing CB-17 SCID mice.

The examples illustrate the invention.

### Example 1: Material and methods

### General

The Fmoc-(9-fluorenylmethoxycarbonyl-) and all other protected amino acid analogs were purchased from Bachem (Bubendorf, Switzerland) or Iris Biotech (Marktredwitz, Germany). Dr. Schottelius from the laboratory of Prof. Wester kindly provided the thioglycosides of galactose, cellobiose and mannose and PSMA I&T was provided by Dr. Wirtz. The tritylchloride polystyrene (TCP) resin was obtained from PepChem (Tubingen, Germany). Chematech (Dijon, France) delivered the chelator DOTAGA-anhydride. All necessary solvents and other organic reagents were purchased from either Alfa Aesar (Karlsruhe, Germany), Sigma-Aldrich (Munich, Germany) or VWR (Darmstadt, Germany). Solid phase synthesis of the peptides was carried out by manual operation using an Intelli-Mixer syringe shaker (Neolab, Heidelberg, Germany). Analytical reversed-phase high performance liquid chromatography (RP-HPLC) was performed on a Nucleosil 100 C18 (5 µm, 125 x 4.0 mm) column (CS GmbH, Langerwehe, Germany) using a Shimadzu gradient HPLC System (Shimadzu Deutschland GmbH, Neufahrn, Germany). Analysis of the peptides was performed by applying different gradients of 0.1% (v/v) trifluoroacetic acid (TFA) in H₂O (solvent A) and 0.1% TFA (v/v) in acetonitrile (MeCN) (solvent B) with a constant flow of 1 mL/min (specific gradients are cited in the text). The Shimadzu SPD 20 A prominence UV/VIS detector (Shimadzu Deutschland GmbH) was used at 220 nm and 254 nm. Human serum albumin (HSA) binding was determined using a Chiralpak HSA 50 x 3 mm 5µm Analytical Column connected to a Chiralpak HSA 10 x 3 mm 5µm Guard Cartridge (Daicel Chemical Industries) purchased from Chiral Technologies Europe (France). Non-linear regression for the HSA binding was performed using OriginPro 2016G. Both retention times t_{R} as well as the capacity factors K' are cited in the text. Preparative RP-HPLC of the peptides was achieved on a Shimadzu HPLC system using a Multospher 100 RP 18-5 (250 x 20 mm) column (CS GmbH, Langerwehe, Germany) with a constant flow of 5 mL/min. Analytical and preparative Radio RP-HPLC of the radioiodinated reference ligand was performed using a Nucleosil 100 C18 (5 µm, 125 x 4.0 mm) column. Radioactivity was detected through connection of the outlet of the UV-photometer to a Nal(TI) well-type scintillation counter from EG&G Ortec (Munich, Germany). The ⁶⁸Ga-and ¹⁷⁷Lu-labeled compounds were analyzed as published previously (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083; Šimeček, J., et al., A Monoreactive Bifunctional Triazacyclononane Phosphinate Chelator with High Selectivity for Gallium-68. ChemMedChem, 2012. 7(8): p. 1375-1378). ESI-mass spectra were acquired on an expression^{L} CMS mass spectrometer (Advion Ltd., Harlow, UK) and on a Varian 500-MS IT mass spectrometer (Agilent Technologies, Santa Clara, USA). For the HSA binding experiment a Chiralpak HSA 50 x 3 mm 5µm Analytical Column, (Daicel Chemical Industries, LTD) connected to a Chiralpak HSA 10 x 3 mm 5µm Guard Cartridge (Daicel Chemical Industries ; Chiral Technologies Europe) was used. Blood samples were collected from volunteers.

### Synthesis of the binding motif Lys-urea-Glu ((OtBu)KuE(OtBu)₂)

The synthesis of the carboxyl-protected Lys-urea-Glu binding motif (**1**) was synthesized as previously described by solution phase synthesis (Weineisen, M., et al., Synthesis and preclinical evaluation of DOTAGA-conjugated PSMA ligands for functional imaging and endoradiotherapy of prostate cancer. EJNMMI research, 2014. 4(1): p. 1).

### Synthesis of the (OtBu)KuE(OtBu)₂-(S)-Fmoc-Asu-OH conjugate

(S)-Fmoc-Asu(OtBu)-OBzl (**2**): To a solution of 50 mg (0.107 mmol, 1.0 eq) (S)-Fmoc-Asu(OtBu)-OH in N,N-dimethylformamide (DMF) was added 21.8 mg (0.16 mmol, 1.5 eq) of 1-Hydroxy-7-azabenzotriazol (HOAt), 61.0 mg (0.161 mmol, 1.5 eq) 1-[Bis(dimethylamino)methylene]-1H-1,2,3-triazolo[4,5-b]pyridinium 3-oxid hexafluorophosphate (HATU) and 73.2 µL (0.48 mmol, 4.5 eq) N,N-diisopropylethylamine (DIPEA). After 15 min of stirring at RT, 22.2 µL (0.32 mmol, 3.0 eq) of benzyl alcohol was further added and the solution stirred overnight. Finally, the solvent was removed in vacuo. Completion of reaction of **2** was analyzed by RP-HPLC (10% to 90% B in 15 min): t_{R} = 17.1 min; K' = 7.55. Calculated monoisotopic mass for **2** (C₃₄H₃₉NO₆): 557.28; found: m/z = 580.7 [M + Na]⁺.

(S)-Fmoc-Asu-OBzl (**3**): tBu deprotection of the crude product **2** was performed with a stirring mixture (v/v) of 95% TFA and 5% dichlomethane (DCM) at RT for 45 min. After evaporation of the solvent the crude product **3** was purified using preparative RP-HPLC (60% to 80% B in 15 min): t_{R} = 9.3 min; K' = 8.9. Calculated monoisotopic mass for **3** (C₃₀H₃₁NO₆): 501.22; found: m/z = 524.5 [M + Na]⁺.

(OtBu)KuE(OtBu)₂-(S)-Fmoc-Asu-OBzl (**4**): To a solution of 51.8 mg (10.3 µmol, 1.0 eq) **3** in DMF was added 20.9 mg (0.15 mmol, 1.5 eq) N-Hydroxybenzotriazole (HOBt), 36.3 mg (15.5 µmol, 1.5 eq) N,N,N',N'-Tetramethyl-O-(benzotriazol-1-yl)uronium tetrafluoroborate (TBTU) and 79.4 µL (59.7 mg, 0.46 mmol, 4.5 eq) DIPEA. After 15 min stirring, 75.6 mg (15.5 µmol, 1.5 eq) of **1** was added and further stirred for 20 h at RT. The crude product **4** was purified using preparative RP-HPLC (70% to 80% B in 15 min): t_{R} = 8.9 min; K' = 1.97. Calculated monoisotopic mass for **4** (C₅₄H₇₄N₄O₁₂): 970.53; found: m/z = 971.8 [M + H]⁺.

(OtBu)KuE(OtBu)₂-(S)-Fmoc-Asu-OH (**5**): For Bzl deprotection, 57.2 mg (0.065 mmol, 1.0 eq) of **4** was dissolved in 2.0 mL of ethanol (EtOH) and 5.72 mg (9.0 µmol, 0.1 eq) of Palladium on activated charcoal (10%) was added. The flask was purged beforehand with H₂ and the solution stirred under light H₂-pressure (ballon). After 70 min stirring, the crude product was filtered through Celite, the EtOH evaporated in vacuo and the product purified using preparative RP-HPLC (70% to 70.5% B in 15 min): t_{R} = 6.5 min; K' = 0.54. Calculated monoisotopic mass for **5** (C₄₇H₆₈N₄O₁₂): 880.48; found: m/z = 881.8 [M + H]⁺.

### Synthesis of the peptidic scaffold

DOTAGA-iodo-D-Tyr-D-Phe-D-Lys-OH (DOTAGA-(l-y)fk) (**6**) was synthesized through solid phase strategy as previously described (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083; Weineisen, M., et al., Synthesis and preclinical evaluation of DOTAGA-conjugated PSMA ligands for functional imaging and endoradiotherapy of prostate cancer. EJNMMI research, 2014. 4(1): p. 1).

### Conjugation of the peptidic scaffold with (OtBu)KuE(OtBu)₂-(S)-Fmoc-Asu-OH and subsequent deprotection

(OtBu)KuE(OtBu)₂-(S)-Fmoc-Asu-OPfp (**7**): To a solution of 13.6 mg (15.4 µmol, 1.0 eq) of **5** in dry DMF was added 4.77 µL (1.94 mg, 30.8 µmol, 2.0 eq) N,N'-Diisopropylcarbodiimide (DIC) and 5.67 mg (30.8 µmol, 2.0 eq) pentafluorophenol (PFP). After 5 min stirring, 2.49 µL (2.43 mg, 31.0 µmol, 2.0 eq) pyridine was added and the solution was allowed to stir overnight at RT. Completion of reaction of **7** was analyzed by RP-HPLC (10% to 90% B in 15 min): t_{R} = 17.2 min; K' = 7.6. Calculated monoisotopic mass for **7** (C₅₃H₆₇F₅N₄O₁₂): 1046.47; found: m/z = 1069.8 [M + Na]⁺.

DOTAGA-(l-y)fk-(S)-Fmoc-Asu-(OtBu)KuE(OtBu)₂ (**8**): To the crude solution of **7,** 16.1 mg (0.0462 mmol, 3.0 eq) of the peptidic scaffold **6** was drop-wise added and stirred overnight with consecutive evaporation of the solvent in vacuo. Completion of reaction of **8** was analyzed by RP-HPLC (10% to 90% B in 15 min): t_{R} = 12.2 min; K' = 5.1. Calculated monoisotopic mass for **8** (C₉₀H₁₂₇IN₁₂O₂₅): 1902.81; found: m/z = 1904.8 [M + H]⁺.

DOTAGA-(l-y)fk-(S)-Asu(NH₂)-KuE (**9**): Fmoc deprotection was achieved by dissolving the crude product **8** in a mixture of (v/v) 20% Piperidine and 80% DMF for 20 min and concomitant solvent evaporation in vacuo. The consecutive removal of tBu-protecting groups was achieved by dissolving the crude product in TFA for 45 min. After precipitation in diethyl ether, the crude product **9** was purified by RP-HPLC (20% to 35% B in 15 min): t_{R} = 12.0 min; K' = 5.0. Calculated monoisotopic mass for **9** (C₆₃H₉₃IN₁₂O₂₃): 1512.55; found: m/z = 1514.1 [M + H]⁺.

### Synthesis of the penta-fluoro-phenyl (PFP) activated thioglycosides

Synthesis of the PFP activated thioglycoside-derivatives of galactose (**10**), mannose (11) and cellobiose (**12**) was carried out as previously published (Elofsson, M., B. Walse, and J. Kihlberg, Building blocks for glycopeptide synthesis: glycosylation of 3-mercaptopropionic acid and Fmoc amino acids with unprotected carboxyl groups. Tetrahedron letters, 1991. 32(51): p. 7613-7616; Kihlberg, J., M. Elofsson, and L.A. Salvador, [11] Direct synthesis of glycosylated amino acids from carbohydrate peracetates and Fmoc amino acids: Solid-phase synthesis of biomedicinally interesting glycopeptides, in Methods in Enzymology. 1997, Academic Press. p. 221-245; Schottelius, M., et al., N-Terminal Sugar Conjugation and C-Terminal Thr-for-Thr(ol) Exchange in Radioiodinated Tyr3-octreotide: Effect on Cellular Ligand Trafficking in Vitro and Tumor Accumulation in Vivo. Journal of Medicinal Chemistry, 2005. 48(8): p. 2778-2789) and kindly provided by the laboratory of Prof. Wester.

### Coupling of the PSMA precursor with the glycosides, deacteylation and purification

For the synthesis of the final glycosylated compounds PSMA I&T Galactose (**13**), PSMA I&T Mannose (**14**) and PSMA I&T Cellubiose (**15**) the synthetic procedure was similar for each compound: 3 mg of **9** (2.0 µmol, 1.0 eq) and either 3.6 mg of **10** (6.0 µmol, 3.0 eq), 3.6 mg of **11** (6.0 µmol, 3.0 eq) or 5.3 mg of **12** (6.0 mmol, 3.0 eq) were dissolved in dry DMF. After addition of 1.0 µL DIPEA (6.0 µmol, 3.0 eq), the solution was stirred overnight at RT. The crude solutions were precipitated in ice-cold diethyl ether, centrifuged and the supernatant removed. Deacetylation was accomplished by dissolving the PSMA inhibitors in MeOH containing 0.5 eq of KCN (Herzig, J., et al., Studies in sugar chemistry. 2. A simple method for O-deacylation of polyacylated sugars. The Journal of Organic Chemistry, 1986. 51(5): p. 727-730) and each solution was again stirred overnight at RT. The final products **13, 14** and **15** were purified by RP-HPLC. PSMA I&T Galactose (**13**): RP-HPLC (10% to 40% B in 15 min): t_{R} = 10.5 min; K' = 4.3. Calculated monoisotopic mass for **13** (C₇₂H₁₀₇IN₁₂O₂₉S): 1762.60; found: m/z = 1763.6 [M + H]⁺. PSMA I&T Mannose (**14**): RP-HPLC (10% to 60% B in 15 min): t_{R} = 9.4 min; K' = 3.7. Calculated monoisotopic mass for **14** (C₇₂H₁₀₇IN₁₂O₂₉S): 1762.60; found: m/z = 1764.1 [M + H]⁺. PSMA I&T Cellubiose (**15**): RP-HPLC (10% to 60% B in 15 min): t_{R} = 7.2 min; K' = 2.6. Calculated monoisotopic mass for **15** (C₇₈H₁₁₇IN₁₂O₃₄S): 1924.66; found: m/z = 1926.8 [M + H]⁺.

### Preparation of the radioiodination precursor

(SnBu₃-BA)(OtBu)KuE(OtBu)₂ (**17**) was prepared as previously published (Weineisen, M., et al., and preclinical evaluation of DOTAGA-conjugated PSMA ligands for functional imaging and endoradiotherapy of prostate cancer. EJNMMI research, 2014. 4(1): p. 1; Maresca, K., et al., A series of halogenated heterodimeric inhibitors of prostate specific membrane antigen (PSMA) as radiolabeled probes for targeting prostate cancer. Journal of medicinal chemistry, 2008. 52(2): p. 347-357; Vaidyanathan, G. and M.R. Zalutsky, Preparation of N-succinimidyl 3-[* I] iodobenzoate: an agent for the indirect radioiodination of proteins. Nature protocols, 2006. 1(2): p. 707-713; Dekker, B., et al., Functional comparison of annexin V analogues labeled indirectly and directly with iodine-124. Nuclear medicine and biology, 2005. 32(4): p. 403-413).

### Synthesis of the nonradioactive reference compounds

### ^{nat}Ga compounds

For the preparation of the respective ^{nat}Ga-**13**, ^{nat}Ga-**14** and ^{nat}Ga-**15** complexes, 50 µL of a 2 mM solution of **13, 14** or **15** in water and 50 µL of a 2 mM solution of Ga(NO₃)₃ in water were mixed and heated at 40°C for 30 min. The chelate formation was assessed using RP-HPLC and MS. The resulting 1 mM solution was diluted and used for in vitro IC₅₀ determination and HSA binding.
[^{nat}Ga]PSMA I&T Galactose (^{nat}Ga-**13**): RP-HPLC (10% to 60% B in 15 min): t_{R} = 7.3 min; K' = 2.7. Calculated monoisotopic mass (C₇₂H₁₀₄GaIN₁₂O₂₉S): 1828.51; found: m/z = 1830.1 [M + H]⁺.
[^{nat}Ga]PSMA I&T Mannose (^{nat}Ga-**14**): RP-HPLC (10% to 60% B in 15 min): t_{R} = 8.0 min; K' = 3.0. Calculated monoisotopic mass (C₇₂H₁₀₄GaIN₁₂O₂₉S): 1828.51; found: m/z = 1830.1 [M + H]⁺.
[^{nat}Ga]PSMA I&T Cellubiose (^{nat}Ga-**15**)**:** RP-HPLC (10% to 60% B in 15 min): t_{R} = 7.7 min; K' = 2.9. Calculated monoisotopic mass (C₇₈H₁₁₅GaIN₁₂O₃₄S): 1991.57; found: m/z = 1992.4 [M + H]⁺.

### ^{nat}Lu compounds

^{nat}Lu-**13**, ^{nat}Lu-**14** and ^{nat}Lu-**15** were prepared in a similar procedure as the ^{nat}Ga compounds, but with a 2.5 molar excess of ^{nat}Lu³⁺. The mixtures were heated for 30 min at 95°C and chelate formation was assessed by RP-HPLC and diluted for in vitro investigations.
[^{nat}Lu]PSMA I&T Galactose (^{nat}Lu-**13**): RP-HPLC (10% to 60% B in 15 min): t_{R} = 7.7 min; K' = 2.9. Calculated monoisotopic mass (C₇₂H₁₀₄ILuN₁₂O₂₉S): 1934.52; found: m/z = 1935.4 [M + H]⁺, 968.2 [M + 2H]²⁺
[^{nat}Lu]PSMA I&T Mannose (^{nat}Lu-**14**): RP-HPLC (10% to 60% B in 15 min): t_{R} = 7.4 min; K' = 2.7. Calculated monoisotopic mass (C₇₂H₁₀₄ILuN₁₂O₂₉S): 1934.52; found: m/z = 1936.4 [M + H]⁺, 968.3 [M + 2H]²⁺
[^{nat}Lu]PSMA I&T Cellobiose (^{nat}Lu-**15**): RP-HPLC (10% to 60% B in 15 min): t_{R} = 7.6 min; K' = 2.8. Calculated monoisotopic mass (C₇₈H₁₁₄ILuN₁₂O₃₄S): 2096.57; found: m/z = 1049.8 [M + 2H]²⁺.

### Radiolabeling

### ⁶⁸Ga-labeling

For in vitro and in vivo studies the ⁶⁸Ga-compounds were prepared with in a fully automated system using a ⁶⁸Ge/⁶⁸Ga generator (iTHEMBA Labs, South Africa) connected to a GallElut+ system (SCINTOMICS GmbH, Germany). The radiolabeling was published in previous reports (Weineisen, M., et al., and preclinical evaluation of DOTAGA-conjugated PSMA ligands for functional imaging and endoradiotherapy of prostate cancer. EJNMMI research, 2014. 4(1): p. 1; Notni, J., et al., TRAP, a Powerful and Versatile Framework for Gallium-68 Radiopharmaceuticals. Chemistry-A European Journal, 2011. 17(52): p. 14718-14722; Notni, J., K. Pohle, and H.-J. Wester, Comparative gallium-68 labeling of TRAP-, NOTA-, and DOTA-peptides: practical consequences for the future of gallium-68-PET. EJNMMI research, 2012. 2(1): p. 1) and the experimental details are described in the supplementary info.

### ¹⁷⁷Lu-labeling

The ¹⁷⁷Lu-labeled compounds, for in vitro studies, were prepared as previously described (Sosabowski, J.K. and S.J. Mather, Conjugation of DOTA-like chelating agents to peptides and radiolabeling with trivalent metallic isotopes. Nat. Protocols, 2006. 1(2): p. 972-976) with minor modifications and used without further purification. In short, to a reaction volume of 10 µL 1.0 M NH₄OAc buffer pH = 5.9 was added 0.75 to 1.0 nmol tracer (7.5 to 10 µL), 10 to 40 MBq ¹⁷⁷LuCl₃ (Specific Activity (As) > 3000 GBq/mg, 740 MBq/mL, 0.04 M HCl, ITG, Garching, Germany) and finally filled up to 100 µL with trace-pure Water (Merck, Darmstadt, Germany). The reaction mixture was heated for 30 min at 95°C and the radiochemical purity was determined using Radio-TLC.

### ¹²⁵I-labeling

The radioiodinated reference ligand ([¹²⁵I]I-BA)KuE was radio-synthesized in accordance to a previously published method (Weineisen, M., et al., Synthesis and preclinical evaluation of DOTAGA-conjugated PSMA ligands for functional imaging and endoradiotherapy of prostate cancer. EJNMMI research, 2014. 4(1): p. 1) and fully described in the supplementary info.

### In vitro assays

### Cell culture

PSMA-positive LNCAP cells (200265; Cell Lines Service GmbH) were cultivated in Dulbecco modified Eagle medium/Nutrition Mixture F-12 (1:1) (DMEM-F12, Biochrom) supplemented with 10% fetal calf serum (Biochrom) and kept at 37°C in a humidified 5% CO₂ atmosphere. One day (24h ± 2h) prior to all experiments with LNCaP cells, the cultivated cells were harvested using a mixture of trypsin/ethylendiaminetetraacetate (0.05%/ 0.02%) and phosphate buffered saline and centrifuged. After centrifugation, the supernatant was disposed and the cell pellet resuspended in culture medium. Afterwards, cells were counted with a hemocytometer (Neubauer) and seeded in 24-well plates. IC₅₀ values were determined transferring 150,000 cells/mL per well into 24-well plates, whereas internalization rates were obtained by transferring 125,000 cells/mL into 24-well PLL-coated plates.

### Determination of IC₅₀ and internalization

The determination of binding affinity (IC50) and internalization was performed using LNCaP cells and the respective ^{68/nat}Ga- and ^{177/nat}Lu-compounds. The experiments were conducted in similarity to a previously published protocol with only minor modifications (Weineisen, M., et al., Synthesis and preclinical evaluation of DOTAGA-conjugated PSMA ligands for functional imaging and endoradiotherapy of prostate cancer. EJNMMI research, 2014. 4(1): p. 1) and are found in detail in the supplementary info.

### HSA binding

HSA binding experiments were performed as previously described (Valko, K., et al., Fast gradient HPLC method to determine compounds binding to human serum albumin. Relationships with octanol/water and immobilized artificial membrane lipophilicity. Journal of pharmaceutical sciences, 2003. 92(11): p. 2236-2248). The mobile phase consisted of a binary gradient system with a constant total flow rate of 0.5mL/min. Mobile phase A was a 50 mM pH 6.9 ammonium acetate solution, mobile phase B was 2-Propanol (HPLC grade, VWR, Germany). The gradient, of mobile phase A was 100% from 0 to 3 min and from 3 min to the end of each run, mobile phase B was set 20%. At each experimental day, the column was calibrated with nine reference substances to confirm the performance and non-linear regression. Afterwards, the actual PSMA inhibitors with unknown HSA binding were measured. More details are found in the supplementary info.

### Lipophilicity

The logP values were determined as described (Weineisen, M., et al., Synthesis and preclinical evaluation of DOTAGA-conjugated PSMA ligands for functional imaging and endoradiotherapy of prostate cancer. EJNMMI research, 2014. 4(1): p. 1). The experimental details are described in the supplementary info.

### Blood cell binding

Approximately 1.0 mL human blood samples from EDTA coated vials, were incubated for 15 min with 225 µL of 1.0 nM solutions of either [¹⁷⁷Lu]PSMA I&T Galactose (¹⁷⁷Lu-**13**) or [¹⁷⁷Lu]PSMA I&T (¹⁷⁷Lu-**18**) and 25 µL of either PBS or PMPA solution (100 µM) in reaction vials. Separation of the different blood cells was accomplished by stepwise centrifugation. Erythrocytes were gained after centrifugation at 700 g (1,400 rpm, Biofuge 15, Heraus Sepatech, Osterode, Germany) for 5 min and the pellet was rinsed with 500 µL of ice-cold PBS and again centrifuged. Leukocytes were gained by combination of the supernatant and subsequent centrifugation at 3,100 g (6,200 rpm) with a further washing step with 500 µL of ice-cold PBS and repeated centrifugation. The activities of the pellets (erythrocytes and leukocytes) and the supernatant were measured in a γ-counter. PSMA specific binding was determined by co-incubation with PMPA solution (100 µM).

### In vivo experiments

All animal experiments were carried out in accordance with the general animal welfare regulations in Germany (Deutsches Tierschutzgesetz, approval #55.2-1-54-2532-71-13). For the tumor model, LNCaP cells (approx. 10⁷ cells) were suspended 1:1 in serum-free DMEM-F12 medium and Matrigel (BD Biosciences, Germany) and inoculated onto the right shoulder of male, 6 to 8 weeks old CB-17 SCID mice (Charles River Laboratories, Sulzfeld, Germany). Animals were used after the tumor size reached 4 to 8 mm in diameter for experiments.

### Metabolic stability

25 MBq of [¹⁷⁷Lu]PSMA I&T Galactose (¹⁷⁷Lu-**13**) was injected into the tail vein of a healthy CB17-SCID mouse, which was sacrificed after 60 min. Samples of the urine and blood were immediately taken. Kidneys were frozen with liquid nitrogen, homogenized and extracted with 500 µL PMPA Solution (400 µM in PBS). After centrifugation at 15,000 g for 5 min, the suspension was ultra-filtrated and analyzed by radio-HPLC. The blood sample was centrifuged at 6,000 g for 5 min to obtain the plasma. To remove the proteins from the plasma, ice-cold MeCN was added to the sample and incubated for 10 min at 4°C. After concomitant centrifugation and ultrafiltration, the sample was analyzed by radio-HPLC. The urine sample was used without any further purification.

### µPET imaging

Imaging experiments were conducted using a Siemens Inveon small animal PET and data analyzed by the associated Inveon Research Workplace software. Mice were anaesthetised with isoflurane and the compounds [⁶⁸Ga]PSMA I&T (⁶⁸Ga-**18**) and [⁶⁸Ga]PSMA I&T Galactose (⁶⁸Ga-**13**) (0.2 nmol, 7.0 to 12 MBq) were injected via tail vein. Dynamic imaging was carried out after on-bed injection for 90 min. The static blockade image was obtained, after 1 h p.i. with 15 min acquisition time. Blockade was done by coinjection of 8mg/kg of PMPA. All images were reconstructed using an OSEM3D algorithm without scanner and attenuation correction.

### Biodistribution

Approximately 10.0 to 11.0 MBq (0.2 nmol) of [⁶⁸Ga]PSMA I&T Galactose (⁶⁸Ga-**13**) were injected into the tail vein of LNCaP tumor-bearing male CB-17 SCID mice and sacrificed after 1 h and 3 h (n = 4, respectively). For [⁶⁸Ga]PSMA I&T (⁶⁸Ga-**18**), 6.3 to 7.0 MBq (0.2 nmol) were injected into healthy CB-17 SCID mice (n = 4) and sacrificed after 1 h. Selected organs were removed, weighted and measured in a γ-counter.

### Example 2: Results

### Synthesis

The introduction of a thioglycoside into the linker region was achieved using (S)-Fmoc-Asu(OtBu)-OH as starting point (Scheme 1). In a first step, the free carboxylic group was protected by introduction of benzyl alcohol via HATU/ HOAt and DIPEA with concomitant acidic removal of the *t*Bu protecting group with TFA, yielding 90% of **3** after RP-HPLC purification. After coupling of **3** with the binding motif **1,** benzyl deprotection was achieved with Palladium on activated charcoal (10%) in EtOH under H₂ atmosphere in 92% yield. After RP-HPLC purification, **5** was activated with DIC, PFP and pyridine and condensed with DOTAGA-(l-y)fk, which was synthesized via solid phase peptide synthesis using standard Fmoc strategy according to a previous protocol (Weineisen, M., et al., Synthesis and preclinical evaluation of DOTAGA-conjugated PSMA ligands for functional imaging and endoradiotherapy of prostate cancer. EJNMMI research, 2014. 4(1): p. 1). The crude product **8** was at first Fmoc-deprotected with subsequent *t*Bu removal. After RP-HPLC purification, DOTAGA-(l-y)fk-(S)-Asu(NH₂)-KuE (**9**) was obtained in 41% yield. To obtain the final glycosylated derivatives **13, 14** and **15,** the provided PFP-activated thioglycosides **10, 11** and **12** were coupled with **9** in an identical procedure using DIPEA to increase the nucleophilicity of the free amine group of **9.** Utilization of KCN in MeOH resulted in mild deacetylation of the final compounds. After RP-HPLC purification of **13, 14** and **15,** all glycosylated compounds were obtained in an overall yield of 3 to 10%.

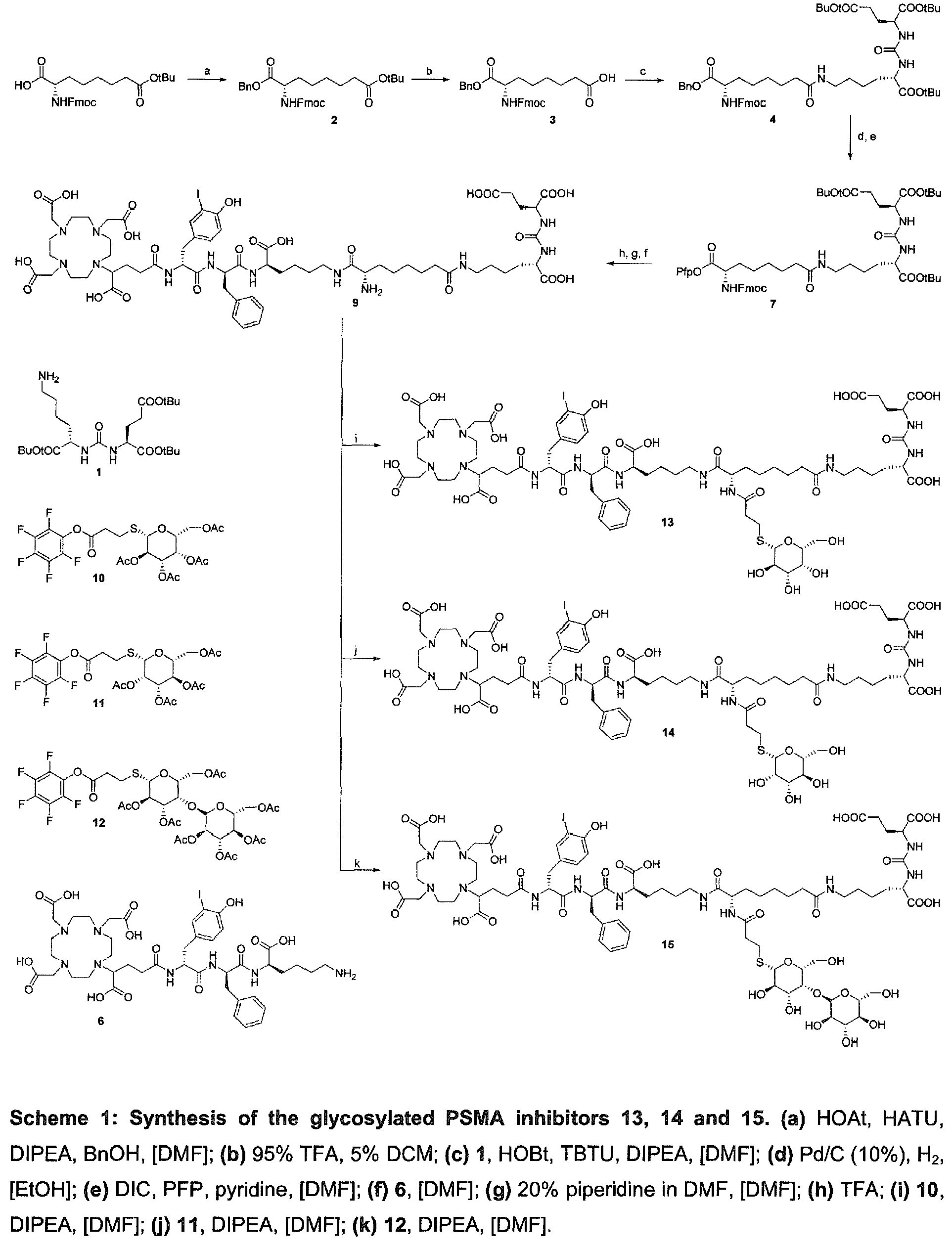

### Radiochemistry

### ⁶⁸Ga-labeling

Readily production of the ⁶⁸Ga-labeled compounds was accomplished within 15 min after start of the radiosynthesis in a fully automated GMP-compliant system. Utilization of 5.0 nmol of the respective PSMA inhibitor yielded in specific activities ranging from 36 to 53 GBq/µmol after cartridge purification. The determined radiochemical purity for all compounds was ≥ 95% and was determined by radio-HPLC. Prior to *in vitro* and *in vivo* applications, the EtOH was evaporated and the ⁶⁸Ga-labeled compounds were diluted with PBS (pH 7.4).

### ¹⁷⁷Lu-labeling

With minor modifications to the previously published protocol (Sosabowski, J.K. and S.J. Mather, Conjugation of DOTA-like chelating agents to peptides and radiolabeling with trivalent metallic isotopes. Nat. Protocols, 2006. 1(2): p. 972-976), the ¹⁷⁷Lu-labeling was accomplished with radiochemical purities ≥ 97% and specific activities ranging from 10 to 53 GBq/µmol. The ¹⁷⁷Lu-labeled compounds were diluted with PBS (pH 7.4) for further applications.

### ¹²⁵I-labeling

For *in vitro* studies ([¹²⁵I]I-BA)KuE was used as reference ligand and synthesized in solution. According to described literature (Weineisen, M., et al., Synthesis and preclinical evaluation of DOTAGA-conjugated PSMA ligands for functional imaging and endoradiotherapy of prostate cancer. EJNMMI research, 2014. 4(1): p. 1; Maresca, K., et al., A series of halogenated heterodimeric inhibitors of prostate specific membrane antigen (PSMA) as radiolabeled probes for targeting prostate cancer. Journal of medicinal chemistry, 2008. 52(2): p. 347-357; Vaidyanathan, G. and M.R. Zalutsky, Preparation of N-succinimidyl 3-[*I] iodobenzoate: an agent for the indirect radioiodination of proteins. Nature protocols, 2006. 1(2): p. 707-713; Dekker, B., et al., Functional comparison of annexin V analogues labeled indirectly and directly with iodine-124. Nuclear medicine and biology, 2005. 32(4): p. 403-413), the respective stannylated precursor **17** was treated with [¹²⁵I]NaI and peracetic acid for 10 min at RT. Consecutive cartridge purification, *t*Bu deprotection with TFA and purification by RP-HPLC resulted in the final product ([¹²⁵I]I-BA)KuE in a radiochemical purity ≥ 99% and a radiochemical yield of 40 ± 10 %. The non-radioactive analog for chromatographic analysis was synthesized by conjugating 4-iodo-benzoic acid to 1 with following *t*Bu deprotection and RP-HPLC purification.

### PSMA affinity (IC₅₀)

Binding to PSMA was determined using LNCaP human prostate cancer cells in a competitive binding assay with the respective ^{nat}Ga or ^{nat}Lu analogs of **13, 14** and **15** and ([¹²⁵I]I-BA)KuE (c = 0.2 nM) as radioligand. For comparison, the *IC₅₀* values of PSMA I&T (**18**) were taken from a previous publication (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083), in which the same binding assay was performed. The results are given in Table 1. Compared to the reference ligand (**18**) and irrespective of the complexed metal ion, the monosaccharide derivatives **13** and **14** displayed higher affinity to PSMA, whereas the introduction of the disaccharide cellubiose (**15**) decreased the affinity to PSMA. The glycosylated compounds, except for **15,** showed the same trend as the reference ligand **18,** in which the ^{nat}Lu-labeled compounds possessed higher affinity as their respective ^{nat}Ga analogs indicating the influence of the free carboxylic acid from the chelator (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083). The different stereochemistry of **13** and **14** had almost no effect on the binding affinity.

**Table 1: PSMA binding affinities (IC₅₀) and internalization [%] of the investigated compounds**

| **PSMA Inhibitor** | | ***IC₅₀* [nM]** | **Internalization [%]** |
|---|---|---|---|
| [^{nat/68}Ga]PSMA I&T | (^{nat/68}Ga-**18**) | 9.4 ± 2.9 | 59.2 ± 1.7 |
| [^{nat/177}Lu]PSMA I&T | (^{nat/177}Lu-**18**) | 7.9 ± 2.4 | 75.5 ± 1.6 |
| [^{nat/68}Ga]PSMA I&T Galactose | (^{nat/68}Ga-**13**) | 7.9 ± 3.9 | 18.7 ± 4.1 |
| [^{nat/177}Lu]PSMA I&T Galactose | (^{nat/177}Lu-**13**) | 5.8 ± 0.6 | 60.2 ± 2.8 |
| [^{nat/68}Ga]PSMA I&T Mannose | (^{nat/68}Ga-**14**) | 7.1 ± 0.3 | 8.4 ± 0.2 |
| [^{nat/177}Lu]PSMA I&T Mannose | (^{nat/177}Lu-**14**) | 5.9 ± 0.5 | 35.3 ± 2.9 |
| [^{nat/68}Ga]PSMA I&T Cellobiose | (^{nat/68}Ga-**15**) | 10.8 ± 1.7 | 4.0 ± 0.6 |
| [^{nat/177}Lu]PSMA I&T Cellobiose | (^{nat/177}Lu-**15**) | 12.5 ± 2.3 | 22.3 ± 1.2 |
| PSMA I Galactose 1 **(21)** | | 3.3 ± 0.6 | - |
| PSMA I Galactose 2 **(22)** | | 13.0 ± 1.4 | - |
| PSMA I Galactose 3 **(23)** | | 3.5 ± 0.3 | - |

Binding assays (*IC₅₀*) were performed using LNCaP cells (150,000/well) and ([¹²⁵I]I-BA)KuE (c = 0.2 nM) as the radioligand. Cells were incubated in HBSS (1% BSA) at 4°C for 1 h. Internalization values were corrected for unspecific binding and normalized to the reference ([¹²⁵I]I-BA)-KuE (c = 0.2 nM for ⁶⁸Ga and 0.5 nM for ¹⁷⁷Lu compounds; 37°C, 1 h, 125,000 cells/well, PLL-coated plates). Data for binding (*IC₅₀*) and internalization are expressed as mean ± SD (n = 3).

### Internalization

LNCaP cells were used to investigate the cell surface binding and internalization of the ⁶⁸Ga-and ¹⁷⁷Lu-labeled compounds **13, 14** and **15.** For all ⁶⁸Ga-labeled compounds, the final peptide concentration was 0.2 nM and 0.5 nM for the ¹⁷⁷Lu-labeled analogs to assure comparability with the obtained data from ⁶⁸Ga-and ¹⁷⁷Lu-**18** (PSMA I&T) (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083). To minimize influence of cell count and cell viability on cell surface binding and internalization, ([¹²¹I]I-BA)KuE (c = 0.2 nM) was likewise measured as reference ligand in all experiments and the obtained data of the radiolabeled compounds **13, 14** and **15** were normalized to the uptake of ([¹²⁵I]I-BA)KuE. Unspecific binding was determined using simultaneous incubation with PMPA (10 µM) and was < 0.8% in all experiments. To differentiate between the cell surface bound and internalized fraction, a PMPA-wash (1.0 µM) was performed to remove the specifically bound radioligand. The cell surface bound fraction after 60 min incubation ranged from 0.6% to 1.0%, whereas the internalized fraction displayed values from 1.3% for ⁶⁸Ga-**15** to 11.3% for ¹⁷⁷Lu-**13** of total applied activity. Normalized to ([¹²⁵I]I-BA)KuE, the results are presented in Table 1. All glycosylated compounds exhibited lower values in comparison to **18** with the tendency of the ⁶⁸Ga-labeled compounds to internalize less by the factor of approximately four than the ¹⁷⁷Lu-analogs. Differences in stereochemistry in **13** and **14** resulted in slightly higher internalization of the galactose derivative. The highest obtained value of the ⁶⁸Ga-labeled compounds was 18.7% ± 4.1% for **13,** which is still more than three times lower than for [⁶⁸Ga]PSMA I&T, exhibiting 59.2% ± 1.7% after 60 min.

### HSA binding

Albumin binding was determined using a HPLC-column with immobilized human albumin for analysis (Valko, K., et al., Fast gradient HPLC method to determine compounds binding to human serum albumin. Relationships with octanol/water and immobilized artificial membrane lipophilicity. Journal of pharmaceutical sciences, 2003. 92(11): p. 2236-2248; Yamazaki, K. and M. Kanaoka, Computational prediction of the plasma protein-binding percent of diverse pharmaceutical compounds. Journal of pharmaceutical sciences, 2004. 93(6): p. 1480-1494). To diminish the influence of temperature and small pH fluctuations of the NH₄OAc buffer on binding (retention time), a set of nine substances with known HSA binding coefficients was used to establish a calibration line by non-linear regression (log retention time vs. log HSA [%] binding). The calibration was always performed prior to the analysis of the actual ^{nat}Ga-and ^{nat}Lu-labeled PSMA inhibitors **13, 14, 15** and **18** and resulted in all experiments in a coefficient of determination (r²) > 92% (supporting info). The results are given in Table 2 and display a range of HSA binding from 6.9% for ^{nat}Ga-**15** to 78.6% for ^{nat}Lu-**18**. For each compound, the values for the ^{nat}Ga-labeled analogs are lower than the ^{nat}Lu-species, indicating the influence of the complexed metal ion and the free carboxylic acid group of the chelator DOTAGA in the case of ^{nat}Ga-labeling. The disaccharide derivative **15** exhibited the lowest measured values irrespective of the metal ion. Comparing **13** and **14,** stereochemistry had only a marginal influence on the albumin binding values, since their respective ^{nat}Ga-and ^{nat}Lu-labeled analogs displayed similar values. Overall, glycosylation reduced the albumin binding in comparison to the reference ligand **18.**

**Table 2: Lipophilicity shown as logP and binding [%] to human serum albumin (HSA) of the labeled PSMA inhibitors**

| **PSMA Inhibitor** | | **log*P*** | **HSA [%]** |
|---|---|---|---|
| [^{68/nat}Ga]PSMA I&T | (^{68/nat}Ga-**18**) | -4.31 ± 0.32 | 48.7 |
| [^{177/nat}Lu]PSMA I&T | (^{177/nat}Lu-**18**) | -4.12 ± 0.1 | 78.6 |
| [^{68/nat}Ga]PSMA I&T Galactose | (^{68/nat}Ga-**13**) | -4.15 ± 0.07 | 7.7 |
| [^{177/nat}Lu]PSMA I&T Galactose | (^{177/nat}Lu-**13**) | -3.95 ± 0.12 | 23.3 |
| [^{68/nat}Ga]PSMA I&T Mannose | (^{68/nat}Ga-**14**) | -4.01 ± 0.08 | 7.6 |
| [^{177/nat}Lu]PSMA I&T Mannose | (^{177/nat}Lu-**14**) | -3.85 ± 0.04 | 25.1 |
| [^{68/nat}Ga]PSMA I&T Cellobiose | (^{68/nat}Ga-**15**) | - | 6.9 |
| [^{177/nat}Lu]PSMA I&T Cellobiose | (^{177/nat}Lu-**15**) | -4.04 ± 0.1 | 19.9 |

| | | | |
|---|---|---|---|
| Data are expressed as mean ± SD (n = 6) for logP. HSA binding [%] was determined via HPLC and non-linear regression calibration using the *t_{R}* of the ^{nat}Ga-and ^{nat}Lu-labeled PSMA inhibitors | | | |

### Lipophilicity

The determination of the log*P* was used to assess lipophilicity. The values for the respective radiolabeled compounds from Table 2 were obtained using the shake flask method (n = 6) in n-octanol and PBS (pH 7.4). Data for ⁶⁸Ga-and ¹⁷⁷Lu-**18** were obtained from a previously published report (Robu, S., et al., Preclinical evaluation and first patient application of 99mTc-PSMA-I&S for SPECT imaging and radioguided surgery in prostate cancer. Journal of Nuclear Medicine, 2016: p. jnumed. 116.178939) and are added in Table 2 for comparison. For all compounds, the respective ¹⁷⁷Lu-labeled analog was more lipophilic than the ⁶⁸Ga-labeled counterpart. In comparison to PSMA I&T (**18**), the introduction of a sugar moiety altered hydrophilicity only to a negligible extent with ⁶⁸Ga-**13** being the most hydrophilic glycosylated PSMA inhibitor displaying a log*P* value of -4.15 ± 0.07.

### Blood cell binding

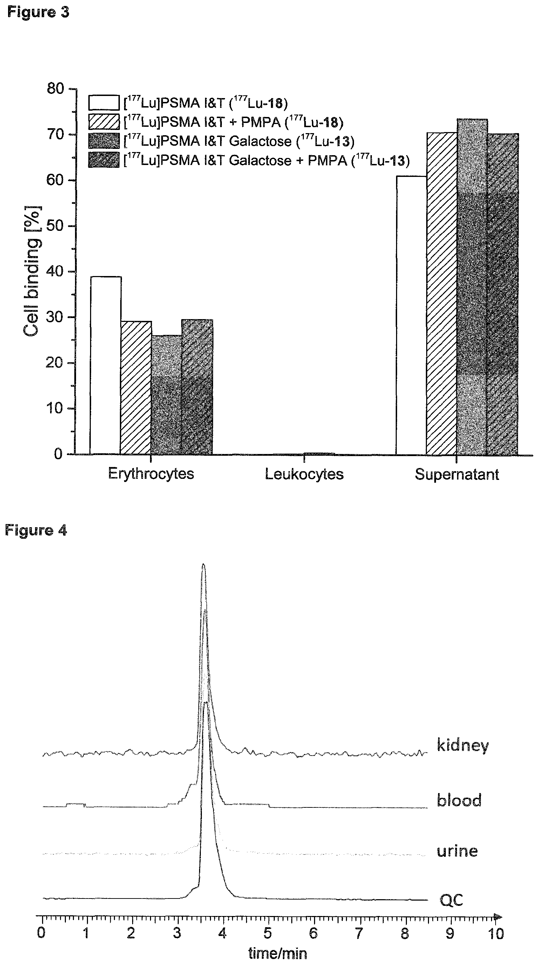

The influence of glycosylation on blood cell binding is shown in Figure 3. **¹⁷⁷Lu-13** and ¹⁷⁷Lu-**18** were incubated in human blood samples and stepwise centrifuged. In addition, 1.0 MBq of the used radiolabeled PSMA ligands were incubated in the presence of PMPA solution (100 µM) to differentiate between specific and unspecific binding. Binding to erythrocytes for ¹⁷⁷Lu-**18** was reduced by simultaneous incubation with PMPA from 38.9% to 29.2%, the resulting difference was not visible in the leukocyte fraction (0.1% vs. 0.2%) but in the supernatant, which increased from 61.1% to 70.7%. Regarding ¹⁷⁷Lu-**13**, incubation with PMPA had only minimal effect on the erythrocyte binding and increased, in contrary to ¹⁷⁷Lu-**18**, the value from 26% to 29.5%. The difference was also found in the supernatant fraction.

### Metabolic stability

Due to the longer half-life of Lutetium-177compared to Gallium-68, the *in vivo* stability of [¹⁷⁷Lu]PSMA I&T Galactose was investigated in a healthy mouse 60 min p.i. and samples extracted from urine, blood and the kidney were analyzed using radio-HPLC as depicted in Figure 4. The extraction rate of ¹⁷⁷Lu-**13** using PMPA as competitor for specific binding was ≥ 31% and enabled reasonable detection. In all analyzed fractions, ¹⁷⁷Lu-**13** found to be stable over 60 min.

### µPET imaging and Biodistribution

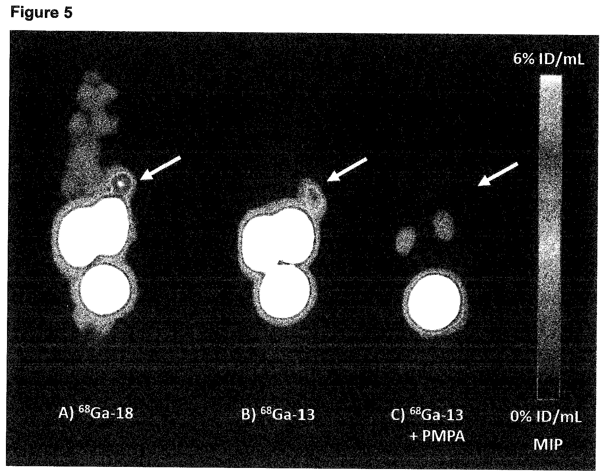

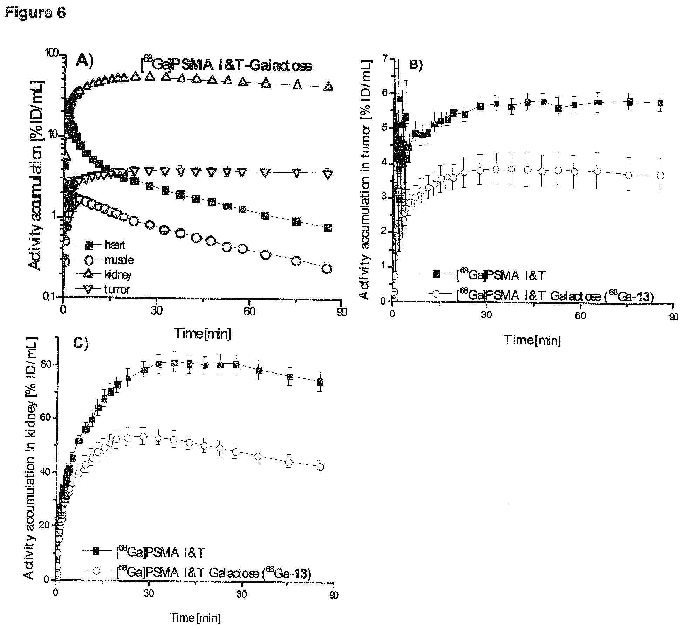

Over a period of 90 min, comparative dynamic µPET scans were performed with ⁶⁸Ga-**13** and ⁶⁸Ga-**18** in CB-17 SCID mice bearing LNCaP tumor xenografts to assess the kinetic tissue distribution. Maximum intensity projections of both tracer are depicted in Figure 5. ⁶⁸Ga-**13** exhibited a general lower uptake in non-PSMA-expressing tissue, but also with less tumor accumulation than ⁶⁸Ga-**18** (3.7 ± 0.5 %ID/mL vs. 5.8 ± 0.3 %ID/mL; 85 min p.i.; Figure 6). Confirmation of specific PSMA-mediated tissue uptake in kidneys and LNCaP tumor of ⁶⁸Ga-**13** was accomplished by coinjection of PMPA (8mg/kg), which reduced the uptake almost completely.

Additionally, the logarithmic plot of the time-activity curves (TAC) in Figure 6 showed a linear decrease of the activity, resulting in a rapid clearance of ⁶⁸Ga-**13** from blood and muscle tissue.

The comparative linear TAC plot correlations for tumor and kidney uptake, displayed a lower accumulation of ⁶⁸Ga-**13** in these PSMA-expressing tissues with earlier onset of kinetic elimination (Figure 6). The biodistribution revealed a similar tendency with general lower or equal tissue accumulation 1 h p.i. of ⁶⁸Ga-**13** (Figure 7) compared to ⁶⁸Ga-**18** except for the blood value. Ongoing clearance of ⁶⁸Ga-**13** from the kidneys and other tissues was visible 3 h after injection, whereas tumor uptake remained stable.

## Claims

1. A compound of formula (I), or a pharmaceutically acceptable salt thereof, 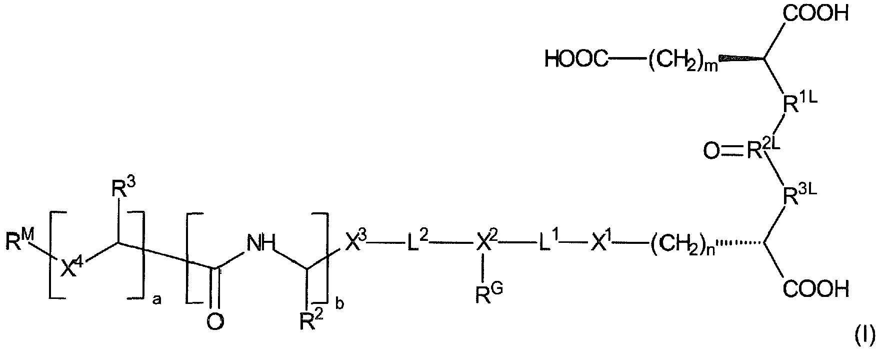 wherein:
m is an integer of 2 to 6;
n is an integer of 2 to 6;
R^{1L} is CH₂, NH or O;
R^{2L} is C or P(OH);
R^{3L} is CH₂, NH or O;
X¹ is selected from an amide bond, an ether bond, a thioether bond, an ester bond, a thioester bond, a urea bridge, and an amine bond,;
L¹ is an alkanediyl group, which alkanediyl group is optionally substituted by a group selected from -COOH and -NH₂;
X² is a trivalent coupling group carrying the substituent R^{G};
L² is an alkanediyl group, said alkanediyl group being optionally substituted by a group selected from -COOH and -NH₂;
X³ is selected from an amide bond, an ether bond, a thioether bond, an ester bond, a thioester bond, a urea bridge, and an amine bond;
a is 0 or 1;
b is 0 or 1;
R² is an optionally substituted aryl group or an optionally substituted aralkyl group, which aryl group or aralkyl group may be substituted on its aromatic ring with a substituent selected from halogen and -OH;
R³ is an optionally substituted aryl group or an optionally substituted aralkyl group, which aryl group or aralkyl group may be substituted on its aromatic ring with a substituent selected from halogen and -OH;
X⁴ is selected from an amide bond, an ether bond, a thioether bond, an ester bond, a thioester bond, a urea bridge, an amine bond, a group of the formula 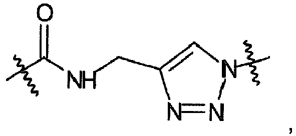 wherein the marked bond at the carbonyl group attaches X⁴ to R^{M} and the other marked bond attaches X⁴ to the remainder of the molecule, and a group of the formula 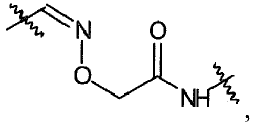 wherein the marked bond at the imino group attaches X⁴ to R^{M} and the other marked bond attaches X⁴ to the remainder of the molecule;
R^{M} is a marker group which comprises at least one of
(i) a chelating group optionally containing a chelated non-radioactive or radioactive cation and
(ii) a radiotracer group containing a covalently bound fluorine atom which can be labeled with ¹⁸F by isotopic exchange of ¹⁹F by ¹⁸F or which is labeled with ¹⁸F; and
R^{G} is a group of the formula (IIa) or (IIb): 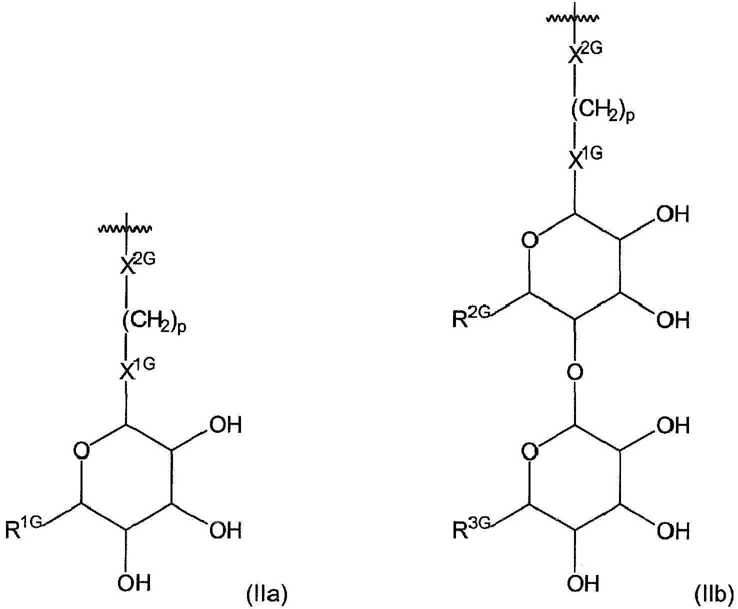
wherein
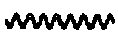 marks the bond which attaches R^{G} to X²;
p is an integer of 1 to 5;
R^{1G} is selected from -OH and -CH₂-OH;
R^{2G} is selected from -OH and -CH₂-OH;
R^{3G} is selected from -OH and -CH₂-OH;
X^{1G} is selected from S and O;
X^{2G} is selected from an amide bond, an ether bond, a thioether bond, an ester bond, a thioester bond, an urea bridge, and an amine bond.

2. The compound or salt of claim 1, wherein m is 2, n is 2 or 4, R^{1L} is NH, R^{2L} is C, and R^{3L} is NH.

3. The compound or salt of claim 1 or 2, wherein X¹ and X³ are both amide bonds.

4. The compound or salt of any of claims 1 to 3, wherein 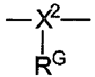 is a group of formula 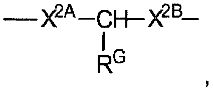 wherein
X^{2A} is absent or is selected from an amide bond, an ether bond, a thioether bond, an ester bond, a thioester bond, an urea bridge, and an amine bond;
X^{2B} is absent or is selected from an amide bond, an ether bond, a thioether bond, an ester bond, a thioester bond, an urea bridge, and an amine bond;
R^{G} is as defined in claim 1;
and wherein X^{2A} attaches X² to L², and X^{2B} attaches X² to L¹.

5. The compound or salt of claim 4, wherein 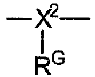 is a group of formula (IIIa) or (IIIb): 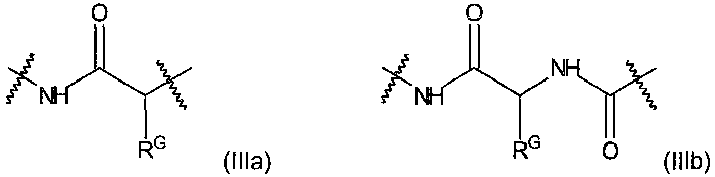 wherein
R^{G} is as defined in claim 1;
and wherein the bond marked with 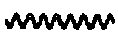 at the N atom attaches X² to L², and the other marked bond attaches X² to L¹.

6. The compound or salt of any of claims 1 to 5, wherein -X³-L²-X²(R^{G})-L¹-X¹- is a group of the formula
-C(O)-NH-CH(COOH)-(CH₂)₄-NH-C(O)-CH(R^{G})-(CH₂)₅-C(O)-NH-
with the C-terminal being attached to the carbon atom in formula (I) carrying the substituent R², and the N-terminal being attached to the group (CH₂)ₙ in formula (I), and with R^{G} being defined as in claim 1.

7. The compound or salt of any of claims 1 to 6, wherein R^{G} is selected from the groups of formula (IIc) and (IId): 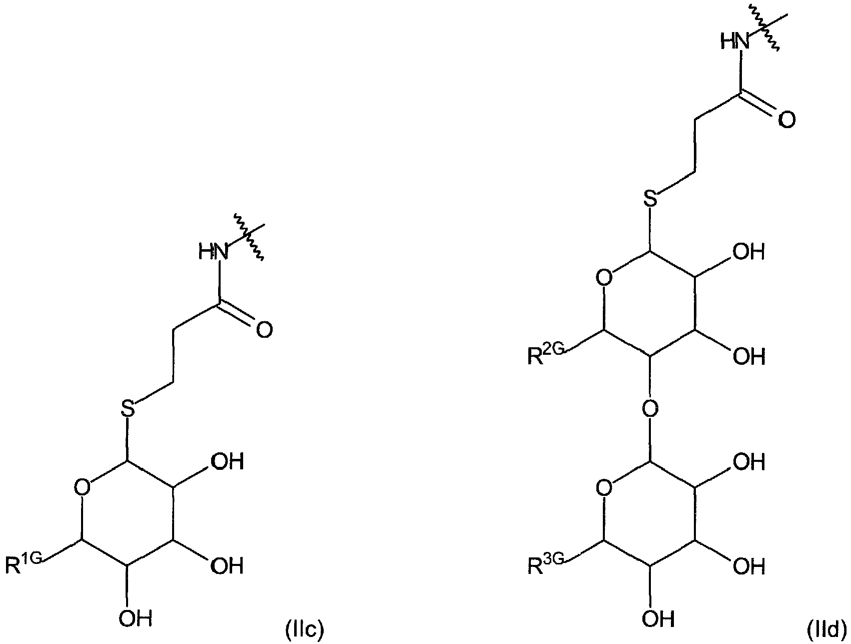 wherein
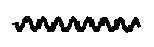 marks the bond which attaches R^{G} to X²; and
R^{1G} is selected from -OH and -CH₂-OH;
R^{2G} is selected from -OH and -CH₂-OH; and
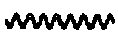 R^{3G} is selected from -OH and -CH₂-OH.

8. The compound or salt of any of claims 1 to 7, wherein R² is an aralkyl group of the formula -CH₂-phenyl or -CH₂-(2-naphthyl), wherein the phenyl and the naphtyl group are optionally substituted with a substituent selected from halogen and -OH.

9. The compound or salt of any of claims 1 to 8, wherein R³ is an aralkyl group of the formula -CH₂-phenyl, wherein the phenyl group is substituted with at least one substituent selected from -I and -OH.

10. The compound or salt of any one of claims 1 to 9, wherein the chelating group comprises at least one of
(i) a macrocyclic ring structure with 8 to 20 ring atoms of which 2 or more are selected from oxygen atoms, sulfur atoms and nitrogen atoms; and
(ii) an acyclic, open chain chelating structure with 8 to 20 main chain atoms of which 2 or more are heteroatoms selected from oxygen atoms, sulfur atoms and nitrogen atoms.

11. The compound or salt of any of claims 1 to 9, wherein X⁴ is a group of the formula 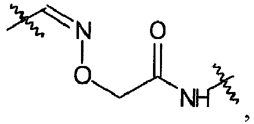 wherein the marked bond at the imino group attaches X⁴ to R^{M} and the other marked bond attaches X⁴ to the remainder of the molecule, and R^{M} is a radiotracer group containing a covalently bound fluorine atom which can be labeled with ¹⁸F by isotopic exchange of ¹⁹F by ¹⁸F or which is labeled with ¹⁸F.

12. The compound or salt of any of claims 1 to 11, which has the formula (Ia): 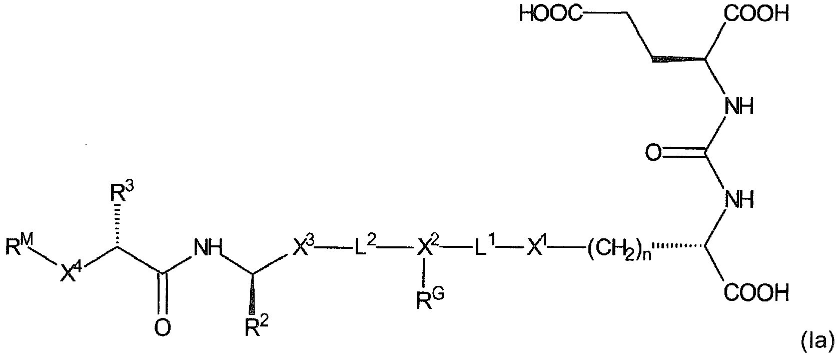 wherein n, X¹, L¹, X², R^{G}, L², X³, R², R³, X⁴ and R^{M} are defined as in the preceding claims.

13. The compound of any one of the preceding claims, wherein said compound is one of the following:

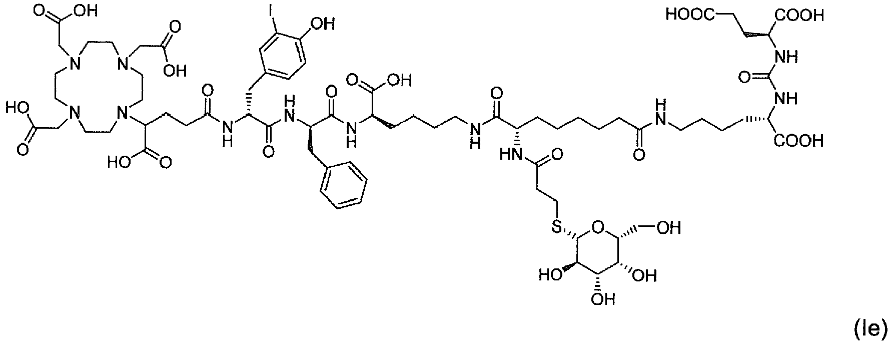

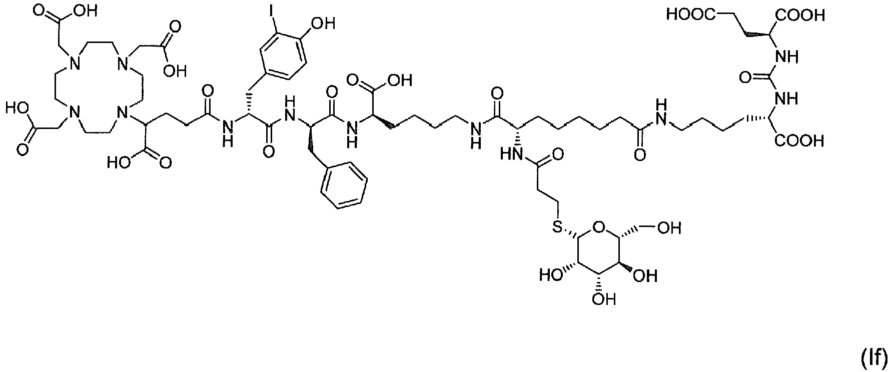

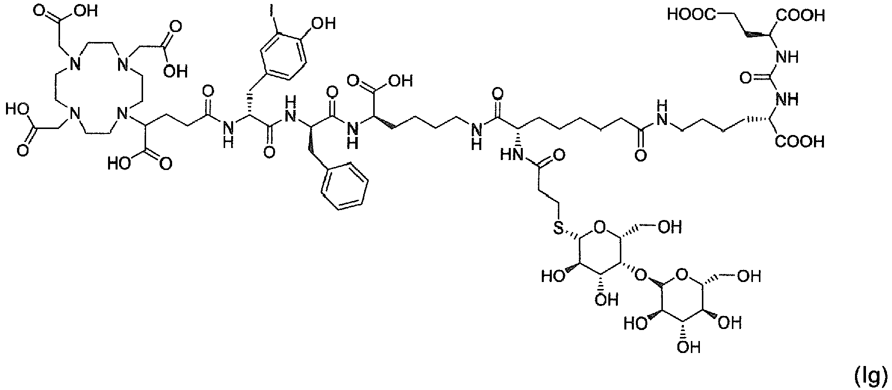

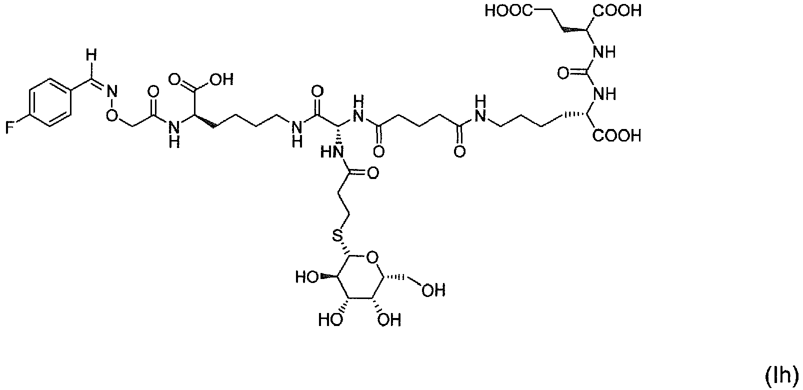

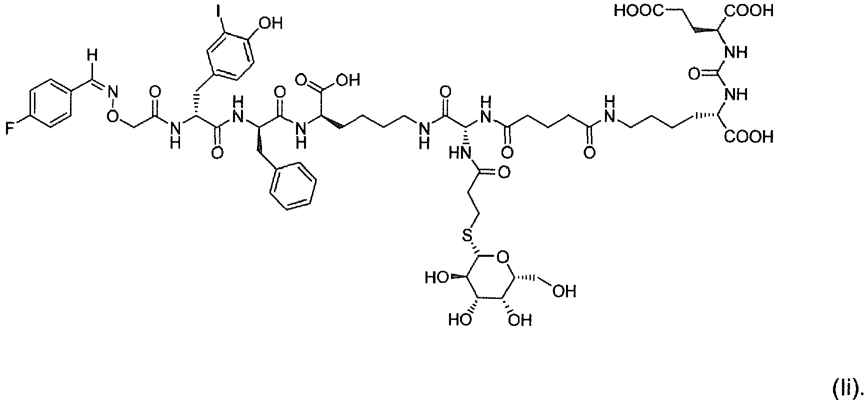

14. A pharmaceutical or diagnostic composition comprising or consisting of one or more compounds or salts in accordance with any one of the preceding claims.

15. A compound or salt in accordance with any one of claims 1 to 13 for use in a method of diagnosing and/or treating
(a) cancer including prostate cancer; or
(b) neoangiogenesis/angiogenesis.
